(19) Europäisches Patentamt
European Patent Office
Office européen des brevets

(11) Publication number: **0 007 973**
**B1**

(12) **EUROPEAN PATENT SPECIFICATION**

(45) Date of publication of patent specification: **01.02.84**

(21) Application number: **79101307.1**

(22) Date of filing: **01.05.79**

(51) Int. Cl.³: **C 07 D 487/04,**
C 07 D 205/08 //C07F7/10,
(C07D487/04, 205/00,
209/00)

(54) **Process for the preparation of thienamycin and intermediates.**

(30) Priority: **14.08.78 US 933323**

(43) Date of publication of application:
**20.02.80 Bulletin 80/4**

(45) Publication of the grant of the patent:
**01.02.84 Bulletin 84/5**

(84) Designated Contracting States:
**AT BE CH DE FR GB IT LU NL SE**

(56) References cited:
**EP - A - 0 017 970**
**US - A - 4 122 086**

**JOURNAL OF THE AMERICAN CHEMICAL SOCIETY, vol. 100, no. 1, January 4, 1978, D.B.R. JOHNSTON et al. "Total synthesis of (+,-)-Thienamycin", pages 313-315**
**Houben-Weyl, Methoden der organischen Chemie (1955) Vol. IX pages 103 and 114**

(73) Proprietor: **MERCK & CO. INC.**
**126, East Lincoln Avenue P.O. Box 2000**
**Rahway New Jersey 07065 (US)**

(72) Inventor: **Christensen, Burton Grant**
**195 Watchung Terrace**
**Scotch Plains, N.J. 07060 (US)**
Inventor: **Salzmann, Thomas Norse**
**387 Brook Avenue**
**North Plainfield, N.J. 07062 (US)**
Inventor: **Ratcliffe, Ronald William**
**234 Matawan Avenue**
**Matawan, N.J. 07747 (US)**

(74) Representative: **Abitz, Walter, Dr.-Ing. et al,**
**Abitz, Morf, Gritschneder P.O. Box 86 01 09**
**D-8000 München 86 (DE)**

**EP 0 007 973 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Process for the preparation of thienamycin and intermediates

This invention relates to the total synthesis of the known antibiotic thienamycin (I).

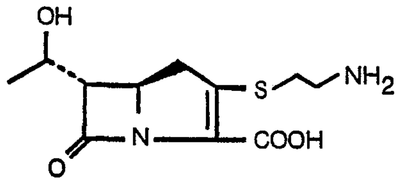

I

and its pharmaceutically acceptable salt and ester derivatives;
comprising reacting:

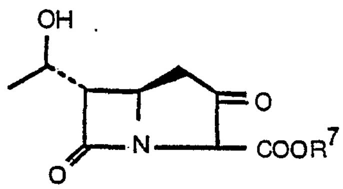

wherein $R^7$ is hydrogen or a protecting group with an acylating agent or a halogenating agent as an activating agent; reacting the obtained compound:

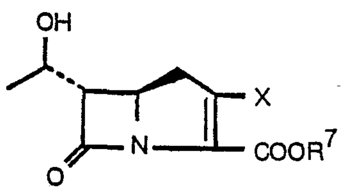

wherein X is a leaving group
with $HSCH_2CH_2NHR^8$;
wherein $R^8$ is hydrogen or a protecting group; and removal of the protecting groups $R^7$ and $R^8$.

In the above process, X is a conventional leaving group, such as halo or organosulfonyloxy, and $R^7$ is hydrogen, a conventional, readily removable protecting group, such as an ester, or a salt cation. The details of the total synthesis are given below.

Including the preparation of starting compounds, the process of the present invention may conveniently be summarized by the following reaction diagram, starting from L-aspartic acid:

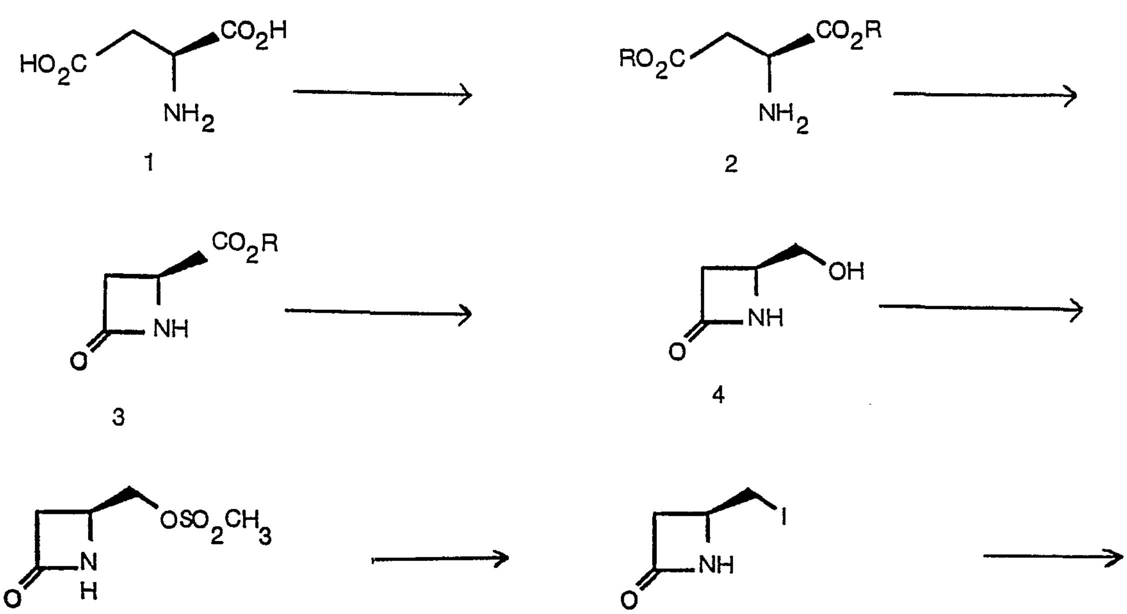

1 2

3 4

4a 5

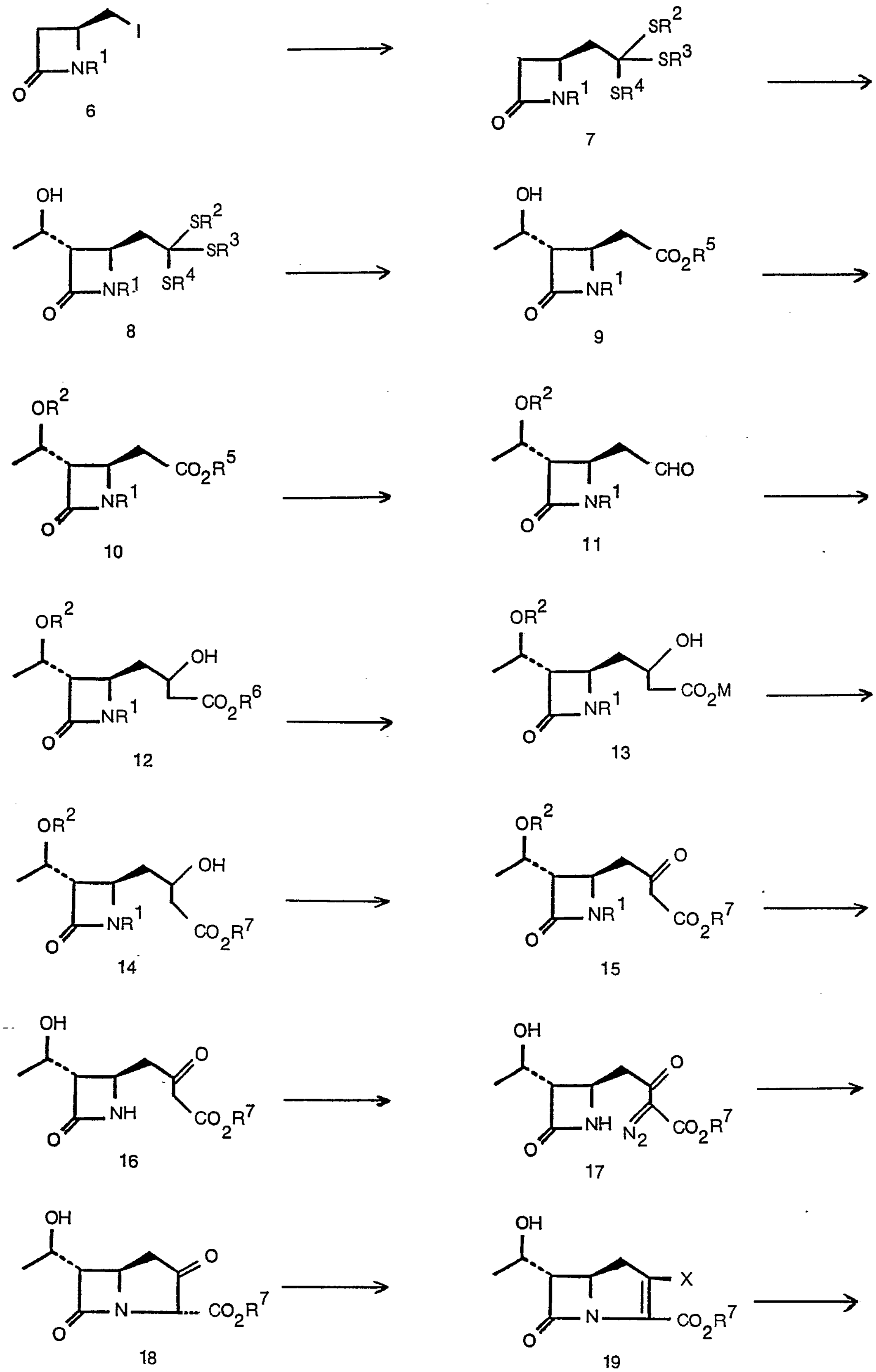

6 7 8 9 10 11 12 13 14 15 16 17 18 19

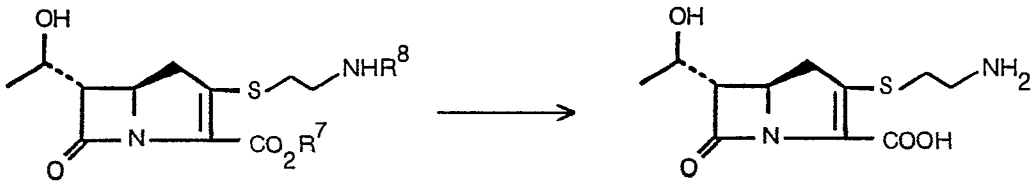

20 I

In words relative to the above diagram, L-aspartic acid 1 is esterified according to well known procedures. Typically 1 in a solvent such as benzene, toluene or chloroform is treated with an esterifying agent such as benzyl alcohol, methanol, ethanol or isopropanol, in the presence of for example p-toluene sulfonic acid, HCl, HBr, at a temperature of from 0 to 110°C for from 1 to 24 hours to achieve the desired establishment and hence protection of the carboxyl functions. The resulting species 2 in a solvent such as ether, THF or DME is treated with for example trimethylchlorosilane, followed by treatment with for example EtMgBr, MeMgl, ØMgBr or t-BuMgCl, at a temperature of from −40 to 50°C for from 1 to 72 hours to provide azetidinone 3. Reduction of species 3 with a reducing agent such as $NaBH_4$, in a solvent such as methanol, ethanol or isopropanol at a temperature of from −10 to 40°C for from 1 to 6 hours provides 4. (For purposes here, the symbols: Et, Me, Ø, iPr, and t-Bu stand for: ethyl, methyl, phenyl isopropyl, and tert-butyl, respectively.)

Treatment of 4 in a solvent such as methylene chloride or $CHCl_3$ with for example, methane sulfonyl chloride or methane sulfonic anhydride in the presence of a base such as $Et_3N$ or $iPr_2NEt$, followed by treatment with a stoichiometric to 5 fold excess of sodium iodide in acetone yields 5 *via* 4a.

The transformation 5 → 6 establishes the protecting group $R^1$ which may be a triorganosilyl group, such as t-butyldimethylsilyl, t-butyldiphenylsilyl, triphenylsilyl, isopropyldimethylsilyl, for example, or may be 3,4-dimethoxybenzyl, for example. Silyl protection is preferred, and typically $R^1$ is established by treating 5 in a solvent such as dimethylformamide, acetonitrile, hexamethylphosphoramide or tetrahydrofuran with a silylating agent such as t-butyldimethylchlorosilane, t-butyldiphenylchlorosilane and triphenylchlorosilane, at a temperature of from −20° to 25°C for from 0.5 to 24 hours in the presence of a base such as triethylamine, diisopropylethylamine, or imidazole.

The transformation 6 → 7 is accomplished by treating 6 in a solvent such as tetrahydrofuran, dimethoxyethane or diethylether with a carbanion generically represented by the following structure:

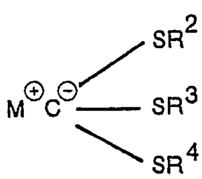

wherein M is a metal cation such as lithium, potassium, copper or magnesium, for example, and $R^2$, $R^3$ and $R^4$ are selected from alkyl, especially having 1 to 6 carbon atoms, aryl or aralkyl, especially having 6 to 10 carbon atoms, such as methyl, ethyl, benzyl, methoxybenzyl, trityl and phenyl, for example, at a temperature of from −100 to 0°C and from 0.5 to 4 hours.

Typically, the carbanion reagent is prepared prior to addition of substrate 6 on treatment of the triorganothiomethane with a strong base such as n-butyllithium, t-butyllithium, phenyllithium or lithium diisopropylamide (LDA).

The alkylation 7 → 8 is accomplished by treating 7 in a solvent such as tetrahydrofuran, dimethoxyethane, diethylether, hexamethylphosphoramide, at a temperature of from −100 to −20°C with a strong base such as lithium diisopropylamide, lithium hexamethyldisilazide, lithium 2,2,6,6-tetramethylpiperidide or potassium hydride followed by the addition of an equivalent to 10 fold excess of acetaldehyde. This reaction gives a mixture of isomers from which the desired *trans*-R form can be conveniently separated by chromatography or crystallization.

The transformation 8 → 9 is accomplished by treating 8 in a solvent such as methanol, ethanol or isopropanol at a temperature of from 0 to 80°C with a Lewis acid such as mercuric chloride, silver tetrafluoroborate or thallium trinitrate. The value of $R^5$ is determined by the identity of the alcohol taken in reaction.

The transformation 9 → 10 establishes the hydroxyl protecting group $R^2$. The most preferred protecting grops $R^2$ are triorganosilyl groups such as t-butyldimethylsilyl, t-butyldiphenylsilyl and triphenylsilyl. Typically, silylation is accomplished by treating 9 with the corresponding triorganosilyl chloride in a solvent such as dimethylformamide, acetonitrile and tetrahydrofuran at a temperature of from −20 to 80°C for from 0.5 to 24 hours.

The reduction 10 → 11 is accomplished by treating 10 in a solvent such as toluene, methylene chloride diethylether and tetrahydrofuran with a reducing agent such as diisobutylaluminum hydride or

sodium bis(2-methoxyethoxy)aluminum hydride at a temperature of from —100° to —40°C for from 1 to 10 hours.

The addition 11 → 12 is accomplished by treating 11 in a solvent such as tetrahydrofuran, diethylether or dimethoxyethane at a temperature of from —100° to 0°C for from 15 minutes to 2 hours in the presence of $LiCH_2CO_2R^6$; wherein $R^6$ is benzyl, p-methoxybenzyl, 2,4-dimethoxybenzyl, for example; which reagent is typically generated *in situ* on treatment of the appropriate $R^6$ acetate with a strong base such as LDA, lithium hexamethyldisilazide or lithium 2,2,6,6-tetramethylpiperide.

If desired, a more readily removable carboxyl protecting group may conveniently replace the first established group, $R^6$, by the carboxyl protecting group $R^7$. This transformation 12 → 13 → 14 is accomplished by selectively deblocking 12 to form 13 by hydrogenation or hydrolysis. Typically, the reaction is accomplished by treating 12 in a solvent such as methanol, ethanol, dioxane, tetrahydrofuran or water under a hydrogenation pressure of from 1 to 4 atmospheres in the presence of a catalyst such as Pd on charcoal or $Pd(OH)_2$, for from 0.1 to 10 hours. The 13 intermediate (M may be H, Na, K or ammonium such as $Et_3NH$, for example) need not be isolated. Intermediate 14 is obtained from the hydrogenation mixture upon treatment with the chosen reagent calculated to establish the protecting group $R^7$ such as an aralkyl halide in a solvent such as dimethylformamide, acetonitrile, hexamethylphosphoramide at a temperature of from 0° to 50°C for from 0.5 to 18 hours. $R^7$ is typically an aralkyl group such as p-nitrobenzyl, or o-nitrobenzyl, for example.

The oxidation 14 → 15 is accomplished by treating 14 in a solvent such as methylene chloride or acetonitrile, with an oxidizing system such as dipyridine chromium (VI) oxide, 3,5-dimethylpyrazole chromium (VI) oxide, pyridinium chlorochromate, pyridinium dichromate, trifluoroacetic anhydride-dimethylsulfoxide or acetic anhydride-dimethyl sulfoxide at a temperature of from —78°C to 25°C for from 5 min. to 8 hrs.

Removal of protecting groups $R^1$ and $R^2$ (15 → 16) is accomplished by acidic aqueous hydrolysis of 15 in a solvent such as methanol, ethanol, tetrahydrofuran or dioxane in the presence of an acid such as hydrochloric, sulfuric or acetic acid at a temperature of from 0 to 100°C for from 2 to 18 hours.

The diazo species 17 is prepared from 16 by treating 16 in a solvent such as $CH_3CN$, $CH_2Cl_2$ or THF with an azide such as p-carboxybenzenesulfonylazide, toluenesulfonylazide or methanesulfonylazide in the presence of a base such as triethylamine, pyridine or $(C_2H_5)_2NH$ for from 1 to 50 hours at 0—25°C.

Cyclization (17 → 18) is accomplished by treating 17 in a solvent such as benzene, toluene or THF at a temperature of from 50—110°C for from 1—5 hours in the presence of a catalyst such as *bis* (acetylacetonato)Cu (II) [$Cu(acac)_2$], $CuSO_4$, Cu powder, $Rh(OAc)_2$, or $Pd(OAc)_2$. Alternatively, the cyclization may be accomplished by irradiating 17 through a pyrex filter (a wave length greater than 300 nm) in a solvent such as benzene, $CCl_4$ or diethylether at a temperature of from 0—25°C for from 0.5 to 2 hours. ["OAc" = acetate.]

Establishment of leaving group X (18 → 19) is accomplished by acylating the keto ester 18 with an acylating agent R°X such as p-toluenesulfonic acid anhydride, p-nitrophenylsulfonic acid anhydride, 2,4,6-triisopropylphenylsulfonic acid anhydride, methanesulfonic acid anhydride, toluenesulfonyl chloride or p-bromophenylsulfonyl chloride, wherein X is the corresponding leaving group such as toluenesulfonyloxy, p-nitrophenylsulfonyloxy, methanesulfonyloxy, p-bromophenylsulfonyloxy and other leaving groups which are established by conventional procedures and are well known in the art. Typically, the above acylation to establish leaving groups X is conducted in a solvent such as methylene chloride, acetonitrile or dimethylformamide, in the presence of a base such as diisopropylethylamine, triethylamine or 4-dimethylamino-pyridine at a temperature of from —20 to 40°C for from 0.5 to 5 hours. The leaving group X of intermediate 19 can also be halogen. The halogen leaving group is established by treating 18 with a halogenating agent such as $Ø_3PCl_2$, $Ø_3PBr_2$, $(ØO)_3PBr_2$ or oxalyl chloride in a solvent such as $CH_2Cl_2$, $CH_3CN$ or THF, in the presence of a base such as diisopropylethylamine, triethylamine, or 4-dimethylaminopyridine. [Ø = phenyl.]

The reaction 19 → 20 is accomplished by treating 19 in a solvent such as dioxane, dimethylformamide, dimethylsulfoxide, acetonitrile or hexamethylphosphoramide, in the presence of an approximately equivalent to excess of the mercaptan reagent $HSCH_2CH_2NHR^8$ wherein $R^8$ is hydrogen or a readily removable N-protecting group such as p-nitrobenzyloxycarbonyl or o-nitrobenzyloxycarbonyl, in the presence of a base such as sodium hydrogen carbonate, potassium carbonate, triethylamine or diisopropylethylamine, at a temperature of from —40 to 25°C for from 1 to 72 hours. The mercaptan reagent, $HSCH_2CH_2NHR^8$, is typically prepared by treating aminoethylmercaptan in the presence of a base such as sodium bicarbonate or sodium hydroxide, in a solvent such as aqueous diethylether, aqueous dioxane or aqueous acetone, at a temperature of from 0 to 25°C for from 0.5 to 4 hours. The reaction of an an alkyl sulfide with an alkyl or aryl halide or an arylsulfonate is known from Houben-Weyl, Methoden der Organischen Chemie, 1955, Vol. IX, pages 103 and 114, however, nothing is said in that reference about the reaction with sulfides of organic halides having a lactam ring.

The final deblocking step 20 → I is accomplished by conventional procedures such as hydrolysis or hydrogenation. Typically 20 in a solvent such as dioxane-water-ethanol or tetrahydrofuran-aqueous dipotassium hydrogen phosphate-isopropanol is treated under a hydrogen pressure of from about 1 to 4 bar in the presence of a hydrogenation catalyst such as palladium on charcoal or palladium hydroxide,

at a temperature of from 0 to 50°C for from 0.5 to 4 hours to provide I.

In the above-discussed aldol reaction 7 → 8 for introduction of the hydroxyethyl side chain, the scheme proceeds directly to give a mixture of isomers (*trans*-R, *trans*-S, and *cis*-R) from which the desired *trans*-R isomer can be separated chromatographically or by crystallization. An indirect aldol reaction scheme stereo-selectively provides the desired *trans*-R isomer according to the following scheme:

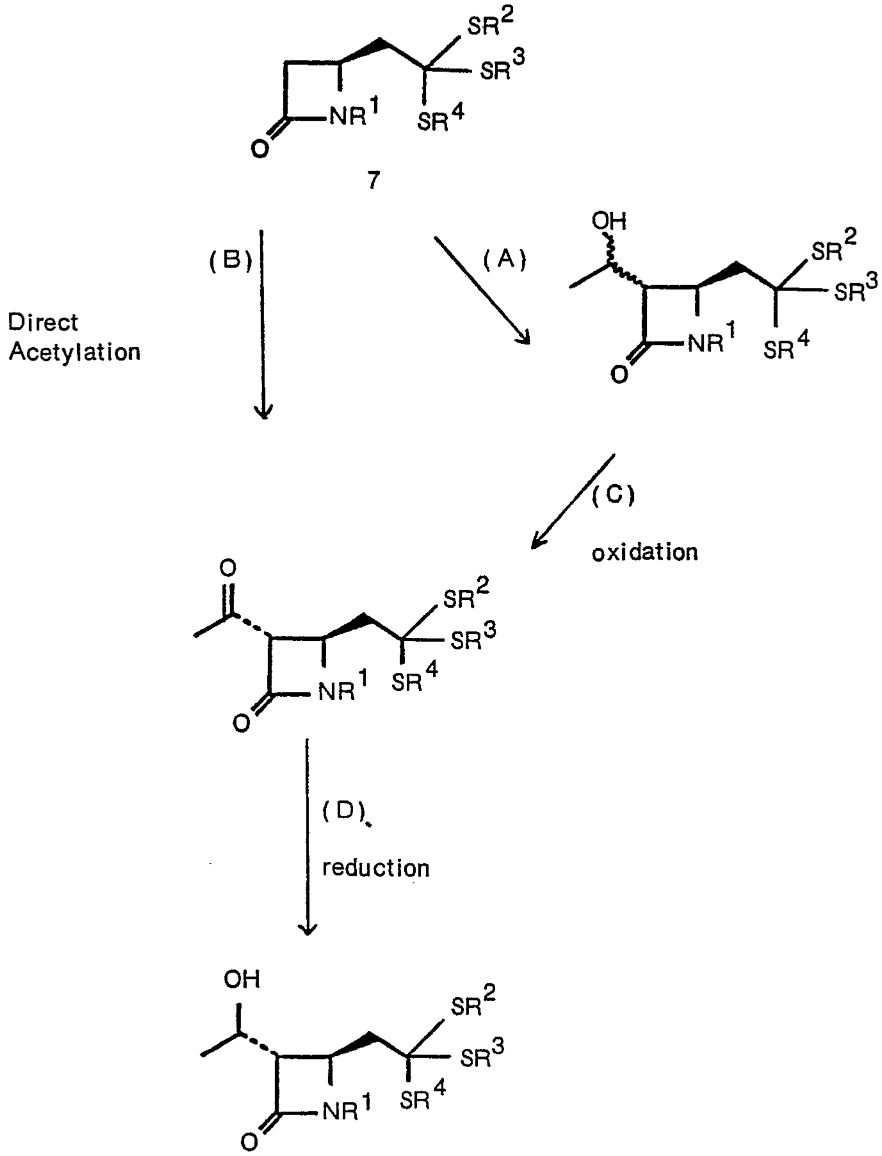


7

8

wherein all symbols are as previously defined.

In words relative to the above reaction scheme, Step A has been described above. The direct acetylation, Step B, is accomplished by treating 7 with two or more equivalents of a base such as lithium diisopropylamide, lithium hexamethyldisilazide, lithium 2,2,6,6-tetramethylpiperidide, in a solvent such as tetrahydrofuran, diethylether, or dimethoxyethane, for example, at a temperature of from —100 to —20°C with an acylating agent such as N-acetyl imidazole. Addition of the 7 plus base mixture to the acylating agent is preferred.

The oxidation Step C is accomplished with an oxidizing agent such as dipyridine chromium(VI)oxide, trifluoroacetic anhydride-dimethylsulfoxide-triethylamine, pyridinium dichromate, acetic anhydride-dimethylsulfoxide in a solvent such as methylene chloride or acetonitrile, at a temperature of from —78 to 25°C for from 5 minutes to 5 hours.

The reduction Step D is accomplished by contacting the ketone with a reducing agent such as potassium tri(sec-butyl)borohydride, lithium tri(sec-butyl)borohydride, sodium borohydride, sodium tris(methoxyethoxy)aluminum hydride or lithium aluminum hydride in a solvent such as diethylether,

tetrahydrofuran or toluene at a temperature of from —20 to 25°C. The reaction can conveniently be conducted in the presence of an added complexing salt such as potassium iodide or magnesium bromide.

In the foregoing word description of the above schematic reaction diagram for the total synthesis of thienamycin, it is to be understood that there is considerable latitude in selection of precise reaction parameters. Suggestion of this latitude and its breadth is generally indicated by the enumeration of equivalent solvent systems, temperature ranges, protecting groups, and range of identities of involved reagents. Further, it is, to be understood that the presentation of the synthetic scheme as comprising distinct steps in a given sequence is more in the nature of a descriptive convenience than as a necessary requirement; for one will recognize that the mechanically dissected scheme represents a unified scheme of synthesis and that certain steps, in actual practice, are capable of being merged, conducted simultaneously, or effected in a reverse sequence without materially altering the progress of synthesis.

The following examples recite a precise scheme of total synthesis. It is to be understood that the purpose of this recitation is to further illustrate the total synthesis and not to impose any limitation.

Example 1

Preparation of 4(S)-4-Iodomethylazetidin-2-one

STEP A

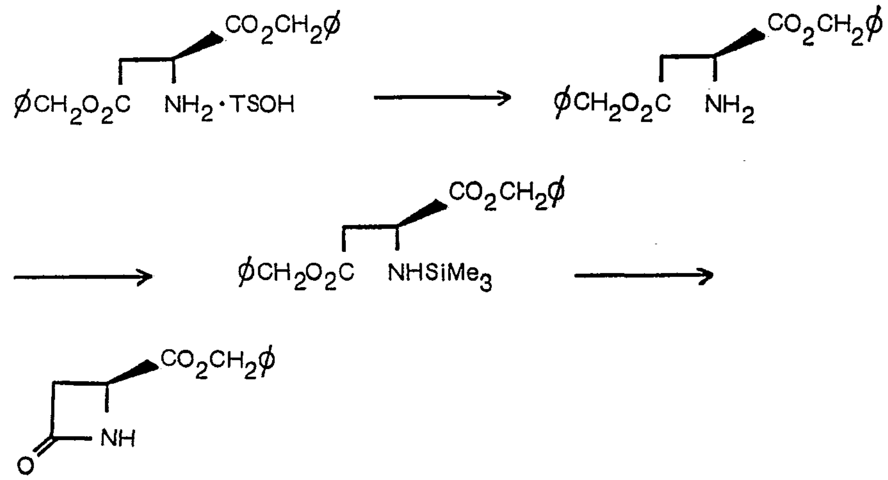

Benzyl (*S*)-azetidin-2-one-4-carboxylate

To a 1000 ml separatory funnel are added dibenzyl (*S*)-aspartate *p*-toluenesulfonic acid salt (48.6 g, 0.1 mole), ice-cold diethyl ether (300 ml), ice-cold water (100 ml), and ice-cold saturated aqueous potassium carbonate (50 ml). The mixture is shaken vigorously and the layers are separated. The aqueous portion is extracted with more cold diethyl ether (2 x 100 ml). The combined ether solution is washed with brine, dried with magnesium sulfate, and evaporated under vacuum to provide dibenzyl (*S*)-aspartate (31.4 g, 0.1 mole) as a colorless liquid.

The dibenzyl (*S*)-aspartate in anhydrous diethyl ether (200 ml) is cooled in an ice-bath under a nitrogen atmosphere. Trimethylchlorosilane (12.7 ml, 0.1 mole) is added to the stirred solution to give a white precipitate. Triethylamine (14.0 ml, 0.1 mole) is then added to the mixture. The cooling bath is removed and the mixture is stirred at room temperature (22—25°C) for 2 hrs. The mixture is then filtered directly into a 3-neck, 1.0 liter, round bottom flask fitted with a sintered glass funnel, magnetic stirrer, and a vacuum-nitrogen inlet. This operation is carried out under a blanket of nitrogen, care being taken to exclude atmospheric moisture. The sintered glass funnel is replaced by a stopper and the ether is evaporated under vacuum with stirring to provide dibenzyl (*S*)-N-trimethylsilylaspartate (35.5 g, 0.092 mole) as a slightly hazy oil.

Anhydrous diethyl ether (250 ml) is added to the flask containing the silyl derivative and the magnetic stirrer is replaced by a mechanical stirrer. The resulting solution is stirred under a nitrogen atmosphere with ice-bath cooling. Ethereal ethyl magnesium bromide (34 ml of a 2.94 M solution, 0.1 mole) is added dropwise over 40 min. to give a cream colored, stirable precipitate. The cooling bath is removed and the mixture is stirred at room temperature. After 1.5 hrs, a viscous gum forms. The mixture is allowed to stand overnight at room temperature. The mixture is then cooled in an ice-methanol bath while ammonium chloride saturated 2N hydrochloric acid (100 ml) is added slowly with stirring. The resulting mixture is diluted with ethyl acetate (100 ml) and water (100 ml) and the layers are separated. The aqueous portion is extracted with more ethyl acetate (3 x 100 ml). The combined organic solution is washed with water (200 ml), 5% aqueous sodium bicarbonate solution (100 ml), water (100 ml), and brine, dried with magnesium sulfate, and filtered. Evaporation of the solvent under vacuum gives an orange oil interspersed with a fine, granular precipitate (25.3 g). This material is

dissolved in warm chloroform (75 ml), diluted with petroleum ether (125 ml), seeded, scratched, and cooled in an ice-bath. The precipitate is collected, washed with petroleum ether, and dried under vacuum to give benzyl (*S*)-azetidin-2-one-4-carboxylate (3.85 g) as an off-white solid mp 136—139°C. The mother liquors and washings are combined, diluted with petroleum ether to 500 ml, seeded, and left in a refrigerator for several days. The resulting precipitate is collected, washed with petroleum ether, and dried under vacuum to give additional product (0.82 g) as pale yellow crystals. Recrystallization of a sample from chloroform-petroleum ether gave the product as small, white flakes: mp 141—143°; $[\alpha]_D = -43.4°$ (c3.275 in $CHCl_3$); IR($CHCl_3$) 3425, 1778, 1746 $cm^{-1}$; $^1H$ NMR($CDCl_3$) $\delta$ 3.00 (ddd, 1, J = 1.9, 3.2, and 14.6 Hz, H-3a), $\delta$ 3.35 (ddd, 1, J = 1.5, 5.4, and 14.6 Hz, H-3b), $\delta$ 4.20 (dd, 1, J = 3.2 and 5.4 Hz, H-4, $\delta$ 5.22 (s, 2, $OCH_2Ph$), $\delta$ 6.48 (m, 1, NH), 7.38 (s, 5, phenyl); mass spectrum m/e 205 (M+), 163, 91, 70, 43.

| | | | | |
|---|---|---|---|---|
| *Anal:* | Calcd. for $C_{11}H_{11}NO_3$: | C, 64.38; | H, 5.40; | N, 6.83. |
| | Found: | C, 64.10; | H, 5.70; | N, 6.77. |

STEP B

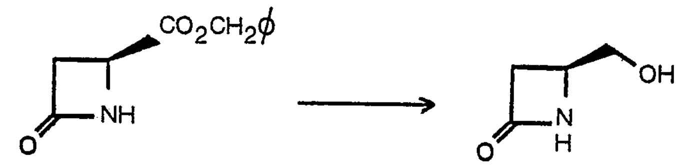

4(*S*)-4-Hydroxymethylazetidin-2-one

Sodium borohydride (3.69 g, 97.5 mmol) is added in one portion to a suspension of benzyl 4(*S*)-azetidin-2-one-4-carboxylate (20.0 g, 97.5 mmol) in 300 ml of absolute methanol at 0°C. The mixture is then allowed to warm slowly with periodic cooling being supplied to keep the internal temperature <30°C. After stirring for 2 hr., glacial acetic acid (23.4 g, 390 mmol) is added and the reaction mixture is concentrated under vacuum. The residue is treated with 500 ml of chloroform and filtered. The filtrate is concentrated under vacuum and the residue is chromatographed on 250 g of silica gel (4:1, chloroform: methanol) to yield 9.62 g (98%) of 4(*S*)-hydroxymethylazetidin-2-one as a white solid: m.p. 51—53°C; $[\alpha]_D = +68.0°$ (C = 2.676 in $CHCl_3$); IR ($CHCl_3$) 3410, 1765 $cm^{-1}$ 1H NMR ($CDCl_3$) $\delta$ 7.07 (1H, br. s, N*H*), $\delta$ 4.05 (1H, br. s, O*H*), $\delta$ 3.77 (2H, m H4, H-H5a or b), $\delta$ 3.58 (1H, dd, J = 11, 6, H-5a or b), $\delta$ 2.97 (1H, ddd, J = 14.5, 4.8, 1.3, H3b), $\delta$ 2.7 (1H, br. d, J = 14.5, H3a); mass spectrum m/e 101 (M+), 83.

STEP C

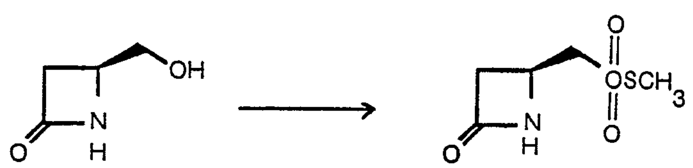

4(*S*)-4-Methanesulfonyloxymethyl azetidin-2-one

Methane sulfonyl chloride (11.46 g, 100 mmol) is added dropwise by syringe to a solution of 4(*S*)-4-hydroxymethyl azetidin-2-one (10.1 g, 100 mmol) and triethyl amine (10.1 g, 100 mmol) in 15 ml of dry methylene chloride at 0°C. (Warming is necessary in order to initially solubilize the alcohol. The resulting solution is then cooled to 0°C prior to addition of the other reagents). The resulting solution is stirred at 0°C for 1 hr. during which time a voluminous precipitate is produced. At the end of this time, the reaction mixture is filtered and the filtrate is concentrated under vacuum. The two solid residues are combined and treated with 500 ml of chloroform. The resulting mixture is filtered to yield substantially pure 4(*S*)-4-methanesulfonyloxymethyl azetidin-2-one as a white solid. The filtrate, which contains most of the triethylamine hydrochloride, is concentrated under vacuum and chromatographed on 200 g of silica gel (4:1 chloroform:methanol) to yield an additional quantity of mesylate. This material is combined with that obtained previously and recrystallized from chloroform to yield 15.57 g (87%) of 4-(*S*)-4-methanesulfonyloxymethylazetidin-2-one as colorless needles: m.p. 109.5—110.5°C; $[\alpha]_D = +25.8°$ (C = 1.025 in $H_2O$);

NMR ($D_2O$) $\delta$ 4.62 (1H, dd, J = 11.2, 3.0, H-5a or b), $\delta$ 4.43 (1H, dd, J = 11.2, 6, H-5a or b), $\delta$ 4.12 (1H, m, H4) $\delta$ 3.26 (3H, s

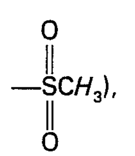

$\delta$ 3.19 (1H, dd, J = 15, 4.5, H3b).

δ 2.88 (1H, dd, J = 15, 2.5, H3a); mass spectrum m/e 179 (M+), 136;

| | | | | |
|---|---|---|---|---|
| *Anal:* Calc: | C, 33.51; | H, 5.06; | N, 7.82; | S, 17.89 |
| Found: | C, 33.54; | H, 5.08; | N, 7.72; | S, 17.93 |

STEP D

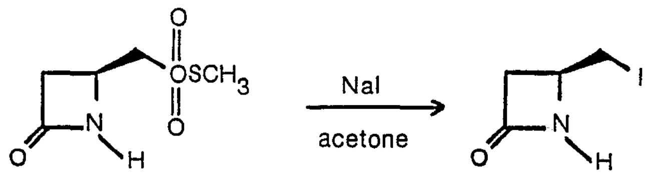


4(*S*)-4-Iodomethylazetidin-2-one

A mixture of 4(*S*)-4-methanesulfonyloxy azetidin 2-one (11.8 g, 65.9 mmol) and powdered sodium iodide (19.8 g, 132 mmol) in 130 ml of acetone is heated at reflux for 6 hr. The resulting reaction mixture is concentrated *in vacuo*, treated with 200 ml of chloroform and filtered. The filtrate is washed with 2 x 50 ml of water and dried over magnesium sulfate. The organic phase is filtered, concentrated *in vacuo*, and chromatographed on 250 g of silica gel (ethyl acetate) to yield 11.94 g (86%) of 4(*S*)-4-iodomethyl-azetidin-2-one as a white solid. This material is recrystallized from ether-petroleum ether to yield white crystals: mp 91—92°C; $[\alpha]_D = -23.7°$ (C = 1.354 in $CHCl_3$); IR ($CHCl_3$) 3450, 1765 $cm^{-1}$, 1H NMR ($CHCl_3$) δ 6.13 (brs, N-*H*), δ 3.94 (m, 1H, Hc), δ 3.56 (m, 2H, Hd and e), δ 3.16 (ddd, 1H, J = 14.9, 5.4, 2.3, Ha), δ 2.72 (d,d,d, 1H, J = 14.9, 2.1 2, Hb) mass spectrum m/e 211 ($M^+$), 168, 142, 127, 84.

Example 2

Preparation of (4*S*)-1-(t-Butyldimethylsilyl)-4-iodomethylazetidin-2-one

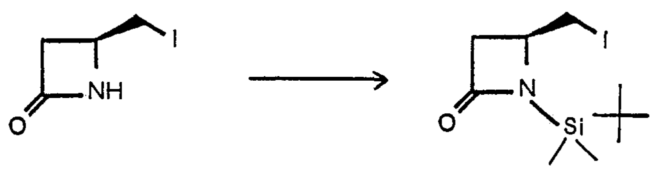

t-butyldimethylchlorosilane (7.51 g, 49.8 mmol) is added in one portion to an ice-cold, stirring solution of (4*S*)-4-iodomethylazetidin-2-one (10.0 g, 47.4 mmol) and triethylamine (5.04 g, 49.8 mmol) in anhydrous dimethylformamide (100 ml). A voluminous white precipitate forms almost immediately. The reaction mixture is stirred at 0—5° for 1 hour and then allowed to warm to room temperature. Most of the solvent is removed under vacuum to give a residue which is partitioned between diethyl ether (250 ml) and water. The ethereal phase is washed with 2.5N hydrochloride acid (50 ml), water (3 x 50 ml), and brine, dried with magnesium sulfate, filtered, and evaporated under vacuum to provide (4*S*)-1-(t-butyldimethylsilyl)-4-iodomethyl-azetidin-2-one (15.1 g) as a white solid. Recrystallization from petroleum ether—ethyl ether gives the product as colorless plates, mp 71—72°; n.m.r. ($CDCl_3$), δ 3.8 (m, 1), δ 2.6—3.6 (2 overlapping d of AB, 4) δ 1.0 (s, 9), δ 0.3 (S, 6), δ 0.25 (s, 6).

Example 3

Preparation of 4(S)-1-(t-Butyldimethylsilyl)-4-(2,2,2-tri(methylthio)-ethyl)azetidin-2-one

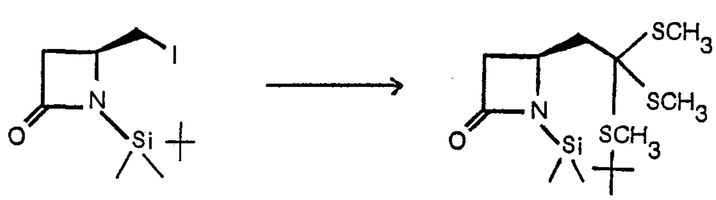

n-Butyllithium (19.4 ml of 2.5M hexane solution, 48.5 mmol) is added slowly by syringe to a solution of tris(methylthio)methane (7.47 g, 48.5 mmol) in 150 ml of freshly distilled THF at —78°C. The resulting solution is stirred at —78°C for 30 min. prior to the addition of a solution of (4*S*) - 1- (tert - butyldimethylsilyl) - 4 - iodomethylazetidin - 2 - one (15.0 g, 46.15 mmol) in 50 ml of THF. This solution is stirred at —78°C for 30 min., then quenched by addition of saturated aqueous ammonium chloride solution. The reaction mixture is allowed to warm to room temperature, then poured into ether (250 ml), washed with water (2 x 100 ml) brine (100 ml) and dried over magnesium sulfate. Removal of solvents *in vacuo* gives an oil which is crystallized from petroleum ether to give 13.3 g (82%) of (4S) - 1 - (t - butyldimethylsilyl) - 4 - (2,2,2 - tri(methylthio)ethyl)azetidin - 2 - one as

colorless prisms. m.p. 61—62°C. IR($CHCl_3$,$CM^{-1}$) 2918, 2850, 1730; n.m.r. ($CDCl_3$) $\delta$ 4.0 (m, 1), $\delta$ 3.35 (dd, 1, J = 5.5, 16), $\delta$ 2.83 (dd, 1, J = 3.16) $\delta$ 2,5 (ABq, 2) $\delta$ 2.15 (s, 9), $\delta$ 0.98 (s, 9), $\delta$ 0.25 (s, 6).

Example 4

Preparation of (3S, 4R)-1-(t-butyldimethylsilyl)-3-[(R)-1-hydroxyethyl]-4-[2,2,2-tri(methylthio)ethyl]-azetidin-2-one

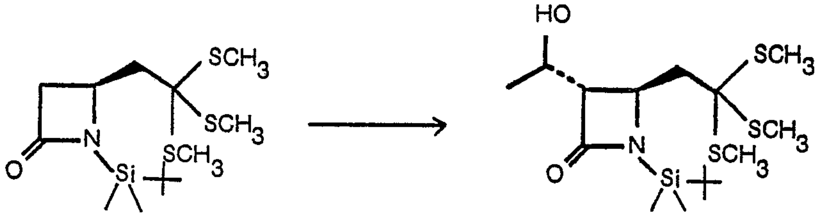

n-Butyllithium (14.8 ml of 2.5N hexane solution, 37.0 mmol) is added by syringe to a solution of diisopropylamine (3.74 g, 37.0 mmol) in 180 ml of freshly distilled tetrahydrofuran at —78°C. The resulting solution is stirred at —78°C for 15 min prior to the addition of a solution of (4S) - 1 - (t - butyldimethylsilyl) - 4 - [2,2,2 - tri(methylthio)ethyl]azetidin - 2 - one (12.34 g, 35.16 mmol) in 35 ml of tetrahydrofuran. This solution is stirred at —78°C for 10 min prior to the addition of acetaldehyde (4.62 g, 105 mmol). The solution is stirred for an additional 5 min. at —78°C and then quenched by addition of saturated aqueous ammonium chloride solution, and allowed to warm to room temperature. The mixture is poured into 250 ml of ether and washed with water (2 × 100 ml) and brine and dried over magnesium sulfate. Removal of solutions *in vacuo* gives an oil which is chromatographed on a silica gel column (1:1 ether: petroleum ether) to give (3*S*,4R) - 1 - (t - butyldimethylsilyl) - 3 - [(R) - 1 - hydroxyethyl] - 4 - [2,2,2 - tri(methylthio)ethyl]azetidin - 2 - one (7.0 g, 50.4%) at $R_f = 0.2$. The product can be recrystallized from petroleum ether. Alternatively, the trans R product can be isolated from the crude reaction mixture by direct crystallization from a petroleum ether solution.

Example 5

Preparation of (3S,4R)-1-(t-Butyldimethylsilyl)-3-(1-oxoethyl)-4-[2,2,2-tri(methylthio)ethyl]azetidin-2-one

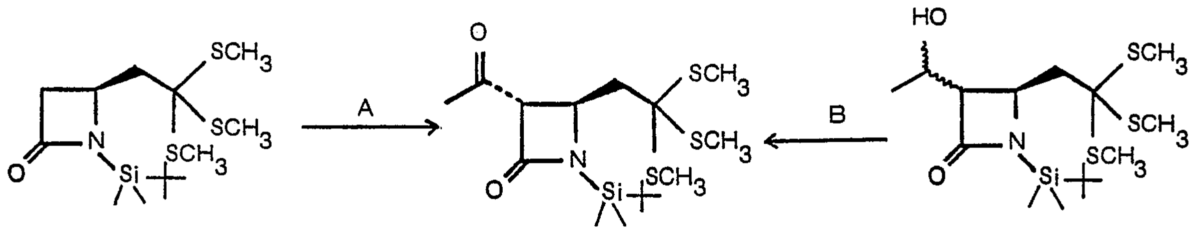


*A.* n-Butyllithium (2.43 ml of 2.4m solution, 5.84 mmol) is added by syringe to a solution of diisopropylamine (591 mg, 5.84 mmol) in 25 ml of freshly distilled tetrahydrofuran at —78°C. The resulting solution is stirred at —78°C for 15 minutes prior to the addition of a solution of (4*R*) - 1 - (t - butyldimethylsilyl) - 4 - [2,2,2 - tri(methylthio)ethyl]azetidin - 2 - one (1.00 g, 2.85 mmol) in tetrahydrofuran (5 ml). This solution is stirred at —78°C for 15 minutes, then added *via* a Teflon tube to a mixture of N-acetylimidazole (642 mg, 5.84 mmol) in 25 ml of THF at —78°C. The resulting yellow reaction mixture is stirred at —78°C for 10 minutes, then quenched by addition of saturated aqueous ammonium chloride solution. The mixture is diluted with ether (200 ml) and washed with 2.5N hydrochloric acid solution (50 ml), water (50 ml) and brine (50 ml) and dried over magnesium sulfate. Removal of solvents *in vacuo* gives a yellow oil which is chromatographed on silica gel (30% ether in petroleum ether) to yield (3*S*, 4*R*) - 1 - (t - butyldimethylsilyl) - 3 - (1 - oxoethyl) - 4 - [2,2,2 - tri(methylthio)ethyl]azetidin - 2 - one. n.m.r. ($CDCl_3$) $\delta$ 4.42 (m, 1), $\delta$ 4.32 (d, 1) $\delta$ 2.35 (m, 2), $\delta$ 2.32 (s, 3), $\delta$ 2.2 (s, 9), $\delta$ 0.98 (s, 9), $\delta$ 0.3 (2s, 6).

*B.* Trifluoroacetic anhydride (400 mg., 1.905 mmol) is added by syringe to a solution of dimethyl sulfoxide (2.53 mmol) in dry methylene chloride (5 ml) at —78°C. The resulting mixture is stirred at —78°C for 30 minutes prior to the addition of a solution of (3RS, 4R) - 1 - (t - butyldimethylsilyl) - 3 - [(RS) - 1 - hydroxyethyl] - 4 - [2,2,2 - tri(methylthio)ethyl] azetidin - 2 - one (500 mg., 1.27 mmol) in dry $CH_2Cl_2$ (1 ml). The resulting solution is stirred for 30 minutes prior to the addition of triethylamine (360 mg., 3.56 mmol). The cooling bath is removed. After 40 minutes, the reaction mixture is diluted with $CH_2Cl_2$, washed with water and brine and dried over magnesium sulfate. Removal of solvents *in vacuo* gives an oil which is purified as above. Yields 432 mg. (86%).

Example 6

Preparation of (3*S*,4*R*)-1-(t-butyldimethylsilyl)-3-[(*R*)-1-hydroxyethyl]-4-[2,2,2-tri(methylthio)ethyl] azetidin-2-one

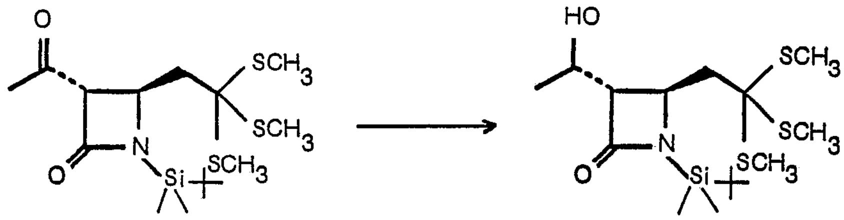

K-Selectride® (3.64 ml of 0.5M, 1.82 mmol) is added by syringe to a mixture of potassium iodide (126 mg., 0.758 mmol) and (3*S*,4*R*) - 1 - (t - butyl dimethylsilyl) - 3 - (1 - oxoethyl) - 4 - [2,2,2 - tri(methylthio)ethyl]azetidin - 2 - one (298 mg, 0758 mmol in freshly distilled ethyl (8 ml) at room temperature. The resulting mixture is stirred at room temperature for 2.5 hours, then quenched by the addition of acetic acid (218 mg., 3.64 mmol). The resulting mixture is diluted with ethyl acetate (25 ml) and filtered through celite. Removal of solvents *in vacuo* gives an oil which is chromatographed on silica gel (ether:petroleum ether) to yield 252 ml of (3*S*, 4*R*) - 1 - (t - butyldimethylsilyl) - 3 - [(*R*) - 1 - hydroxyethyl] - 4 - [2,2,2 - tri(methyltrio) ethyl]azetidin - 2 - one. N.M.R. (*R* isomer, $CDCl_3+D_2O$) $\delta$ 4.15 (dq, 1), $\delta$ 3.95 (ddd, 1, J = 9.5, 2.3), $\delta$ 3.26 (dd, 1, J = 8, 2.3), $\delta$ 2.37 (m, 2), $\delta$ 2.16 (s, 9), $\delta$ 1.37 (d, 3, J = 6,6), $\delta$ 1.0 (s, 9), $\delta$ 0.26 (s, 6).

Example 7

Preparation of (3S,4R)-1-(t-Butyldimethylsilyl)-3-((R)-1-hydroxyethyl)-4-carbomethoxymethylazetidin-2-one

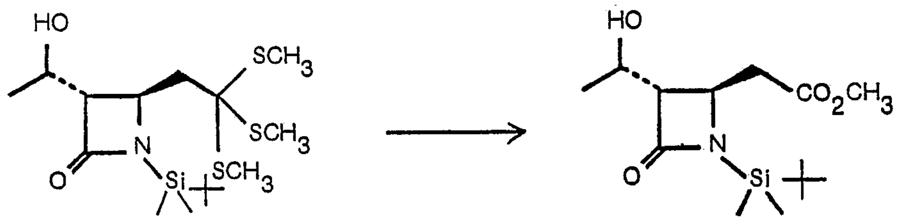

Mecuric chloride (12.37 g, 45.6 mmol) is added in one portion to a solution of (3S,4R) - 1 - (t - butyldimethylsilyl) - 3 - [(R) - 1 - hydroxyethyl] - 4 - [2,2,2 - tri(methylthio)ethyl] azetidin - 2 - one (6.0 g 15.2 mmol) in 250 ml of absolute methanol at 0°C. The resulting mixture (heavy white precipitate) is stirred at 0°C for 3 min., then quenched by addition of sodium bicarbonate (8.99 g, 107 mmol). This mixture is then filtered and the solid residue is washed with additional methanol. The combined filtrate and washings are concentrated *in vacuo* and the residue is partitioned between ethyl acetate and saturated aqueous ammonium chloride solution. The organic phase is separated, washed with saturated aqueous ammonium chloride solution, water and brine and dried over magnesium sulfate. Removals of solvents *in vacuo* gives an oil which is chromatographed on a silica gel column (3:2 cyclohexane: ethyl acetate) to yield (3S, 4R) - 1 - (t - butyldimethylsilyl) - 3 - [(R) - 1 - hydroxyethyl)] - 4 - carbomethoxymethyl azetidin - 2 - one.

Example 8

Preparation of (3S,4R)-1-(t-Butyldimethylsilyl)-3-[(R)-1-(t-butyldimethylsilyloxy)ethyl]-4-carbomethoxymethylazetidin-2-one

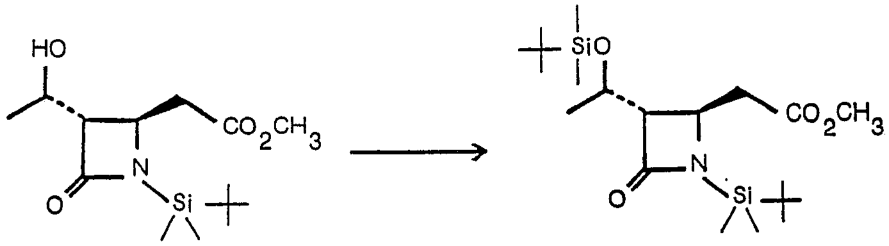

t-Butyldimethylchlorosilane (940 mg, 6.25 mmol) is added in one portion to a solution of (3S, 4R) - 1 - (t - butyldimethylsilyl) - 3 - [(R) - 1 - hydroxyethyl] - 4 - carbomethoxymethylazetidin - 2 - one (1.88 g, 6.25 mmol) and triethylamine (1.27 g, 6.25 mmol) in 15 ml of anhydrous dimethylformamide at 0°C. After 15 min. at 0°C the cooling bath is removed and the reaction mixture is stirred at room temperature for 24 hrs. Ether (100 ml) is added and the mixture is filtered, then washed with 2.5N hydrochloric acid (20 ml), water (3 x 20 ml) and brine. The organic phase is dried

over magnesium sulfate, then concentrated *in vacuo*. The residue is chromatographed on silica gel (7:3 petroleum ether:ether) to yield (3S, 4R) - 1 - (t - butyldimethylsilyl) - 3 - [(R) - 1 - (t - butyldimethylsilyloxy)ethyl] - 4 - carbomethoxymethylazetidin - 2 - one. n.m.r. ($CDCl_3$) δ 4.1 (m, 2), δ 3.68 (S, 3), δ 3.03 (dd, 1, J = 4.3, 2.7), δ 2.8 (ABq, 2), δ 1.17 (d, 3, J = 6.6), δ 0.98 (s, 9), δ 0.89 (s, 9), δ 0.23 (s, 6), δ 0.1 (s, 6).

Example 9

Preparation of (3S,4R)-1-(t-Butyldimethylsilyl)-3-[(R)-1-(t-butyldimethylsilyloxy)ethyl]-4-(2-oxoethyl)azetidin-2-one

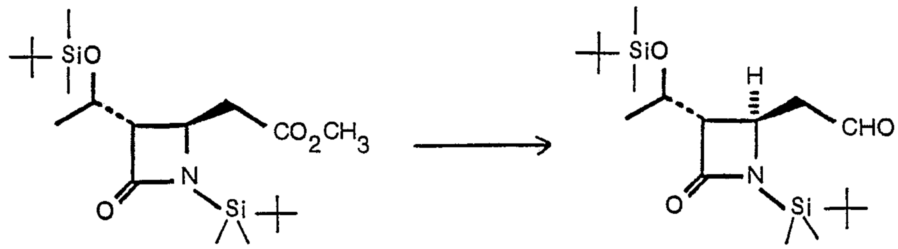

Diisobutylaluminum hydride (3.72 ml of 0.81 M in hexane, 3.38 mmol) is added slowly by syringe to a solution of (3S, 4R) - 1 - (t - butyldimethylsilyl) - 3 - [(R) - 1 - (t - butyldimethylsilyloxy)-ethyl] - 4 - carbomethoxymethyl azetidin-2-one (936 mg, 2.26 mmol) in 25 ml of freshly distilled toluene at −78°C. The resulting solution is stirred at −78°C for 3 hrs., then quenched by addition of 2.5N hydrochloric acid (5 ml). The resulting mixture is stirred for 2 min., then poured into a separatory funnel containing 100 ml of ether and 50 ml of 1.25N hydrochloric acid saturated with tartaric acid. The organic phase is separated and the aqueous phase is extracted with ether (2 x 50 ml). The combined organic phases are washed with brine and dried over magnesium sulfate. Removal of solvents *in vacuo* gives a white solid which is recrystallized from ether-petroleum ether to give (3S,4R) - 1 - (t - butyldimethylsilyl) - 3 - [(R) - 1 - (t - butyldimethylsilyloxy)ethyl] - 4 - (2 - oxoethyl)azetidin - 2 - one. m.p. 115—116°C; n.m.r. ($CDCl_3$) δ 4.1 (m, 1), δ 4.03 (m, 1), δ 2.7—3.2 (m, 3), δ 1.23 (d, 3, J = 6.4), δ 1.08 (s, 9), δ 0.9 (s, 9), δ 0.25 (s, 6), δ 0.1 (s, 6), δ 9.83 (t, 1, J = 1.4).

Example 10

Preparation of (3S,4R)-1-(t-Butyldimethylsilyl)-3-[(R)-1-(t-butyldimethylsilyloxy)ethyl]-4-(3-benzyloxycarbonyl-2-hydroxypropyl)azetidin-2-one

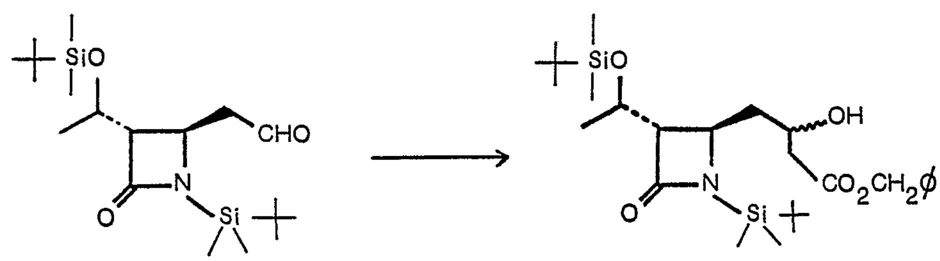

n-Butyllithium (1.81 mmol) is added by syringe to a solution of diisopropylamine (1.81 mmol) in 9 ml of freshly distilled tetrahydrofuran at −78°C. The resulting solution is stirred for 15 min at −78°C. Benzyl acetate (1.81 mmol) is then added dropwise by syringe and the resulting solution is stirred at −78°C for 20 min. A solution of (3S, 4R) - 1 - (t - butyldimethylsilyl) - 3 - [(R) - 1 - (t - butyldimethylsilyloxy)ethyl] - 4 - (2 - oxoethyl)azetidin - 2 - one (1.64 mmol) in 3 ml of anhydrous tetrahydrofuran is added slowly by syringe. The reaction mixture is stirred at −78°C an additional 15 min and then quenched by addition of saturated aqueous ammonium chloride solution. Ethyl acetate (50 ml) is added and the organic phase is separated, washed with water (2 x 10 ml) and brine and dried over magnesium sulfate. Removal of solvents *in vacuo* gives a white solid which is chromatographed on a short silica gel column (40% ether in petroleum ether) to yield (3S,4R) - 1 - (t - butyldimethylsilyl) - 3 - [(R) - 1 - (t - butyldimethylsilyloxy)ethyl] - 4 - (3 - benzyloxycarbonyl - 2 - hydroxypropyl)azetidin - 2 - one. n.m.r. ($CDCl_3$) δ 7.32 (s, 5), δ 5, 1 (s, 2), δ 4.0 (m, 3), δ 2,4—3,8 (m, 4), δ 2.0 (m, 2), δ 1.25 (overlapping d, 3), δ 0.95 (s, 9), δ 0,9 (s, 9), δ 0.3 (s, 6), δ 0.18 (s, 6).

Example 11

Preparation of (3S,4R)-1-(t-Butyldimethylsilyl)-3-[(R)-1-(t-butyldimethylsilyloxy)ethyl]-4-[3-(4-nitrobenzyl)oxycarbonyl-2-hydroxypropyl] azetidin-2-one

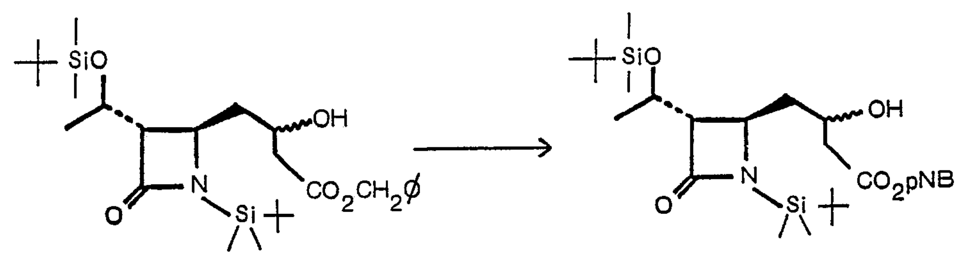

A mixture of (3S,4R) - 1 - (t - butyldimethylsilyl) - 3 - [(R) - 1 - (t - butyldimethylsilyloxy)-ethyl] - 4 - (3 - benzyloxycarbonyl - 2 - hydroxypropyl)azetidin - 2 - one (1.00 mmol), sodium bicarbonate (1.00 mmol) and 10% Pd/C in 20 ml of 4:1 tetrahydrofuran-$H_2O$ is hydrogenated at 40 psi on a Parr shaker for 30 min. The mixture is then filtered through Celite and the catalyst is washed with 10 ml of water. The combined washings and filtrate are concentrated i.v. to 2 ml and lyophilized. The resulting fluffy white solid is dissolved in 5 ml of anhydrous dimethylformamide and p-nitrobenzyl bromide (216 mg, 1.00 mml) is added in one portion. The resulting solution is stirred at room temperature for 3 hrs, then diluted with ether (50 ml) and washed with water (3 x 10 ml) and brine and dried over magnesium sulfate. The solvents are removed *in vacuo* and the residue is chromatographed on silica gel to yield (3S,4R) - 1 - (t - butyldimethylsilyl) 3[(R) - 1 - (t - butyldimethylsilyloxy)-ethyl] - 4 - [3 - (4 - nitrobenzyl)oxycarbonyl - 2 - hydroxypropyl]azetidin - 2 - one. n.m.r. ($CDCl_3$) $\delta$ 7.85 (2d-aromatic, 4), $\delta$ 5,26 (s, 2), $\delta$ 4.2 (m, 3), $\delta$ 2,5—3.6 (m, 4) $\delta$ 2.0 (m, 2), $\delta$ 1.4 (2 overlapping d, 3), $\delta$ 1.0 (2s, 18), $\delta$ 0.25 (2s, 12).

Example 12

Preparation of (3S,4R)-1-(t-Butyldimethylsilyl)-3-[(R)-1-(t-butyldimethylsilyloxy)ethyl]-4-[3-(4-nitrobenzyl)oxycarbonyl-2-oxopropyl]azetidin-2-one

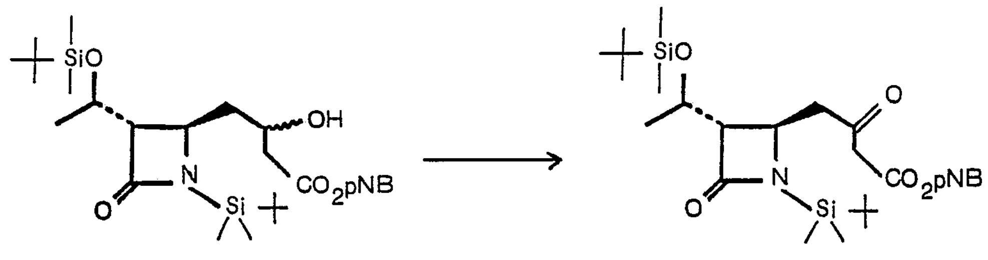

Anhydrous chromium trioxide (10.0 mmol) is added to a solution of anhydrous pyridine (20.0 mmol) in 30 ml of anhydrous methylene chloride. After stirring at room temperature for 15 min., the reaction mixture is treated all at once with a solution of (3S,4R) - 1 - (t - butyldimethylsilyl) - 3 - [(R) - 1 - (t - butyldimethylsilyloxy)ethyl - 4 - [3 - (4 - nitrobenzyl)oxycarbonyl - 2 - hydroxypropyl]azetidin - 2 - one (1.00 mmol) in anhydrous methylene chloride (8 ml). The resulting mixture is stirred at room temperature for 5 min. The $CH_2Cl_2$ layer is decanted from the dark, tarry residue which is triturated with more $CH_2Cl_2$. The combined $CH_2Cl_2$ phase is concentrated *in vacuo*. The residue is triturated with ether (100 ml) and the ether extracts are filtered. The filtrate is washed with 5% aqueous sodium bicarbonate solution, 2.5N HCl, 5% $NaHCO_3$ and brine, dried over magnesium sulfate and concentrated *in vacuo* to yield (3S,4R) - 1 - (t - butyldimethylsilyl) - 3 - (R) - 1 - (t - butyldimethyl-silyloxy)ethyl] - 4 - [3 - (4 - nitrobenzyl)oxycarbonyl - 2 - oxopropyl]azetidin - 2 - one. n.m.r. ($CDCl_3$) $\delta$ 7.85 (2d-aromatic, 4), $\delta$ 5.27 (s, 2), $\delta$ 4.05 (m, 2), $\delta$ 3.6 (s, 2), $\delta$ 2.4—3.2 (dd overlapping ABq, 3), $\delta$ 1.2 (d, 3, J = 6.6), $\delta$ 0.9 (2s, 18), $\delta$ 0.22 (s, 6), $\delta$ 0.05 (s, 6).

Example 13

Preparation of (3S,4R)-3-[(R)1-hydroxyethyl]-4-[3-(4-nitrobenzyl)oxycarbonyl-2-oxopropyl]azetidin-2-one

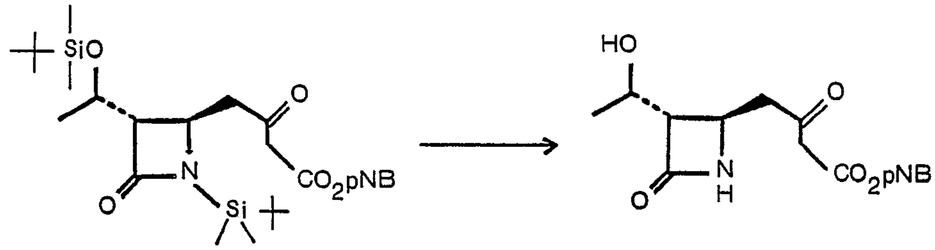

(3S,4R) - 1 - (t - butyldimethylsilyl)3 - [(R) - 1 - (t - butyldimethylsilyloxy)ethyl] - 4 - [3 - (4 - nitrobenzyl)oxycarbonyl - 2 - oxopropyl]azetidin - 2 - one (7.9 mmol) is dissolved in 160 ml of 9:1 (v/v) methanol-water and cooled to 0°C. Concentrated hydrochloric acid (2.75 ml) is added and the resulting solution is stirred at 0°C for 15 min., then allowed to warm to room temperature. The solution is stirred at room temperature for 2.5 hrs, then diluted with ethyl acetate (200 ml) and washed with water, saturated aqueous bicarbonate solution and brine, dried over magnesium sulfate and concentrated *in vacuo* to yield (3S, 4R) - 3 - [(R)1 - hydroxyethyl] - 4 - [3 - (4 - nitrobenzyl)-oxycarbonyl - 2 - oxopropyl] - azetidin - 2 - one.

Example 14

Preparation of (3*S*,4R)-3-[(R)-1-Hydroxyethyl]-4-[3-(4-nitrobenzyl)-oxycarbonyl-2-oxo-3-diazopropyl]azetidin-2-one

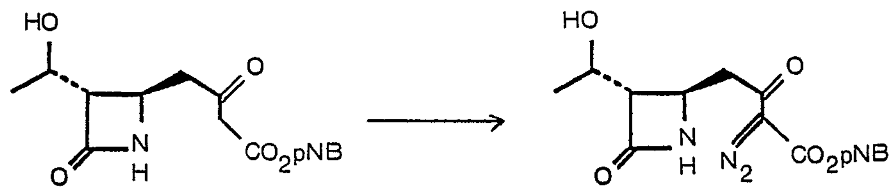

Triethylamine (263 mg, 2.6 mmol) is added by syringe to a mixture of (3*S*, 3*R*) - 3 - [(R) - 1-hydroxyethyl] - 4 - [3 - (4 - nitrobenzyl) oxycarbonyl - 2 - oxypropyl]azetidin - 2 - one (253 mg, [0.72] mmol) and p-carboxybenzene sulfonylazide (196 mg, 0.84 mmol) in dry acetonitrile (6 ml) at 0°C. When addition is complete the cooling bath is removed and the reaction mixture is stirred at room temperature for 1 hour. The mixture is then diluted with ethyl acetate (50 ml) and filtered. The filt rate is concentrated *in vacuo* and the residue is chromatographed on a short silica gel column (ethyl acetate) to yield 222 mg, (81% overall from (3*S*, 4*R*) - 1 - (t - butyldimethylsilyl) - 3 - [(R) - 1 - (t - butyl dimethylsilyloxy)ethyl] - 4 - [3 - (4 - nitrobenzyl)oxycarbonyl - 2 - oxopropyl]azetidin - 2 - one) of (3*S*,4*R*) - 3 - (R) - (1 - hydroxyethyl) - 4 - [3 - (4 - nitrobenzyl)oxycarbonyl - 2 - oxo - 3 - diazopropyl]azetidin - 2 - one as a white solid m.p. (dec.) 163°C. IR($CHCl_3$, $CM^{-1}$) 3410, 2132, 1756, 1718, 1650, 1350, 1280, 1120; n.m.r. ($CDCl_3$) $\delta$ 7.9 (2d-aromatic, 4) $\delta$ 5.4 (s, 2), $\delta$ 6.2 (brs, 1), $\delta$ 4.1 (m, 2), $\delta$ 2.6—3.6 (m, 4), $\delta$ 1.32 (d, 3, J = 6.2).

Example 15

Preparation of (5*R*,6*S*)p-Nitrobenzyl 6-[(*R*)1-hydroxyethyl]-1-azabicyclo[3.2.0]heptan-3,7-dione-2-carboxylate

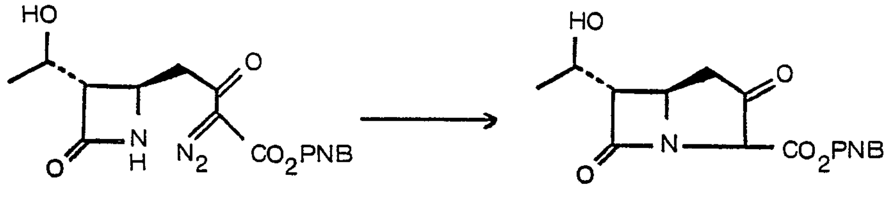

A suspension of (3*S*,4*R*) - 3 - [(R) - 1 - hydroxyethyl] - 4 - [3 - (4 - nitrobenzyl)-oxycarbonyl - 2 - oxo - 3 - diazopropyl]azetidin - 2 - one (56.4 mg, 0.15 mmol) and rhodium (II) acetate (0.1 mg) in dry benzene (3 ml) is deoxygenated by bubbling through nitrogen for 10 minutes. The mixture is then heated to +78°C for 1 hour. During heating the solid starting material gradually goes into solution. The mixture is then cooled, filtered to remove the catalyst, and the filtrate is concentrated *in vacuo* to yield (5*R*, 6*S* p-nitrobenzyl 6 - [(R) - 1 - hydroxyethyl] - 1 - azabicyclo[3.2.0]heptan - 3,7 - dione - 2 - carboxylate, 51 mg. (98%) as a colorless oil which slowly crystallized at room temperature (22°C).

Physical Properties

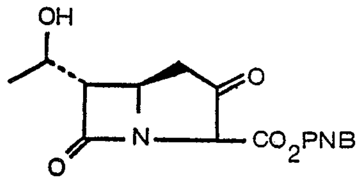

PNB = p-nitrobenzyl

n.m.r.: (300MHz, $CDCl_3$) δ 8.26, 7.54 (aromatic, 4), 5.29 (AB, 2), 4.77 (s, 1), 4.32 (dq, I, J = 6.6, 7), 4.16 (ddd, 1, J = 7, 7.5, 2.2), 3.21 (dd, 1, J = 7, 2.2), 2.94 (dd, 1, J = 19.5, 7) 2.50 (dd, 1, J = 19.5, 7.5), 2.2 (brs, 1), 1.37 (d, 3, J = 6.6).

I.R. ($CHCl_3$,$CM^{-1}$) 1770, 1758, 1610, 1522, 1353 m.p. 110—111°C.

Example 16

Preparation of *p*-Nitrobenzyloxycarbonylaminoethanethiol

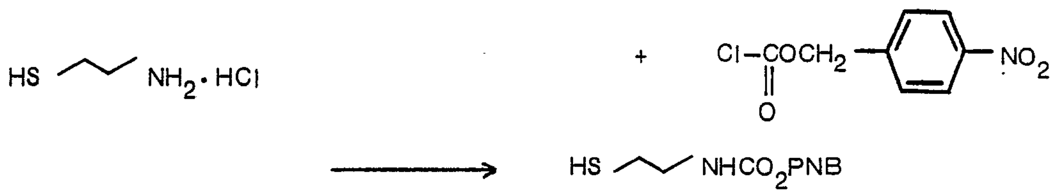

To 600 ml diethyl ether ($Et_2O$)—75 ml $H_2O$ in an ice bath with stirring is added 3.2 g cysteamine hydrochloride (mw = 114; 28.1 mmole). A solution of 7.14 g $NaHCO_3$ (mw = 84; 85 mmole) in 75 ml $H_2O$ is added. The ice bath is removed, and at room temperature a solution of 6.75 g *p*-nitrobenzylchloroformate (mw = 216; 31.3 mmole) in 270 ml $Et_2O$ is added dropwise over a period of one hour. After 10 additional minutes, the layers are separated. The ether layer is extracted with 150 ml 0.25 N HCl, and then with 200 ml brine. Each aqueous layer is then backwashed successively with 100 ml $Et_2O$. The combined $Et_2O$ layers are dried over anhydrous $MgSO_4$, filtered, and concentrated under a $N_2$ stream. The crystalline residue is slurried in a small amount of ether, filtered, and the pale yellow crystals are dried under vacuum to give 4.7 g *p*-nitrobenzyloxycarbonylaminoethanethiol (65% yield). NMR ($CDCl_3$): 8.18 (*d*, J = 8Hz, aromatic protons ortho to nitro), 7.47 (*d*, J = 8Hz, aromatic protons meta to nitro), 5.27 (—N*H*—), 5.20 (*s*, $CH_2$—NH—), 2.67 (*m*, —$CH_2$—SH), 1.35 (*t*, J = 8.5Hz, —S*H*) in ppm downfield from TMS. IR ($CHCl_3$ solution): carbonyl- 1725 $cm^{-1}$. M.S.: molecular ion-256, (M—47) at 209, (M—136) at 120, $^{+}CH_2\varnothing pNO_2$ at 136.

Example 17

Preparation of (5*R*,6*S*) p-Nitrobenzyl 3-[2-(p-nitrobenzyloxycarbonyl)amino ethylthio]-6-[(R)-1-hydroxyethyl]-1-azabicyclo [3,2,0]-hept-2-en-7-one-2-carboxylate

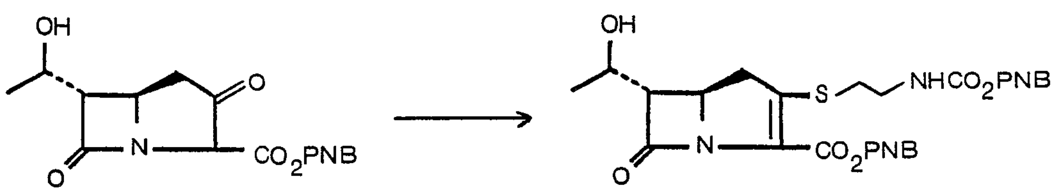

(5*R*,5*S*) p-Nitrobenzyl 6 - [(*R*)1 - hydroxyethyl] - 1 - azabicyclo [3,2,0]heptan - 3,7 - dione - 2 - carboxylate (51 mg, 0.147 mmol) is dissolved in acetonitrile (3 ml) and the resulting solution is cooled to 0°C. Diisopropylethylamine (22 mg, 0.17 mmol) is added by syringe and the resulting solution is stirred at 0°C for 1 minute prior to the addition of a solution of freshly recrystallized p-toluene sulfonic anhydride (51 mg., 0.156 mmol) in dry acetonitrile (1 ml). The resulting solution containing the isolable intermediate (5*R*, 6*S*) p - Nitrobenzyl - 3 - (p - toluene sulfonyloxy) - 6 - [(R) - 1 - hydroxyethyl] - 1 - azabicyclo[3.2.0]hept - 2 - en - 7 - one - 2 - carboxylate is stirred at 0°C for 1 hour, then cooled to −25°C. Diisopropylethylamine (80.5 mg, 0.624 mmol) is added by syringe followed shortly thereafter by a solution of N-p-nitrobenzyloxycarbonylcysteamine (40 mg, 0.156 mmol) in 1 ml of dry acetonitrile. The reaction mixture is then stored in a refrigerator for 70 hr. The mixture is diluted with 25 ml of ethyl acetate washed with brine and dried over magnesium sulfate. Solvents are removed *in vacuo* to yield a yellow oil which is chromatographed on a silica gel plate (ethyl acetate, $R_f$ = 0.4) to yield (5*R*, 6*S*) p - nitrobenzyl - 3 - [2 - (*p* - nitrobenzyloxycarbonyl)amino ethylthio] - 6 - [(R) - 1 - hydroxyethyl] - 1 - azabicyclo [3,2,0] - hept - 2 - en - 7 - one - 2 - carboxylate as

a yellow solid, m.p. 167—169°C. IR(Nujol mull) 1773 and 1690 $cm^{-1}$; n.m.r. ($CDCl_3$) $\delta$ 7.54—8.26 (overlapping ABq, 4), $\delta$ 5.40 (ABq, 2), $\delta$ 5.22 (s, 2), $\delta$ 4.27 (m, 2), $\delta$ 3.47 (m), $\delta$ 3.23 (dd, 1), $\delta$ 3.14 (dd, 1) $\delta$ 3.40 (dd, 1), $\delta$ 3.04 (m, 2), $\delta$ 1.37 (d, 3).

Example 18

Preparation of Thienamycin

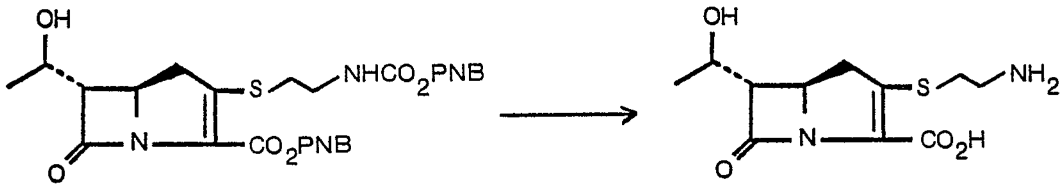

A mixture of N-p-nitrobenzyloxycarbonyl thienamycin p-nitrobenzyl ester (10 mg, 0.017 mmol) and 10% Pd/C-Bolhofer type in tetrahydrofuran (2 ml), 0.1 M dipotassium hydrogen phosphate solution (1.4 ml) and 2-propanol (0.2 ml) is hydrogenated at 40 psi on the Parr shaker for 30 minutes. The mixture is then filtered and the catalyst is washed with water (3 x 3 ml). The combined filtrate and washings are extracted with ethyl acetate-ethyl ether then concentrated to ~3 ml and lyophilized. The resulting white powder is identical to natural thienamycin in all respects.

**Claims for the Contracting States: BE CH DE FR GB IT LU NL SE**

1. A process for preparing:

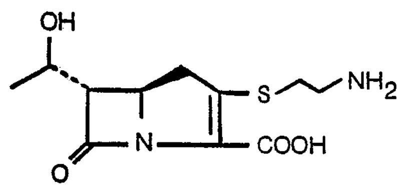

I

and its pharmaceutically acceptable salt and ester derivatives;
comprising reacting:

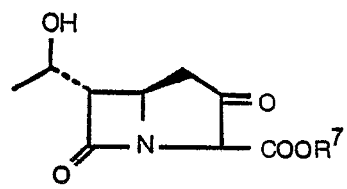

wherein $R^7$ is hydrogen or a protecting group with an acylating agent or a halogenating agent as an activating agent, reacting the obtained compound:

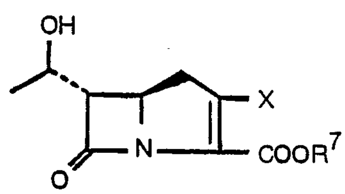

wherein X is a leaving group
with $HSCH_2CH_2NHR^8$;
wherein $R^8$ is hydrogen or a protecting group; and removal of the protecting groups $R^7$ and $R^8$.

2. A process according to Claim 1, wherein the starting compound

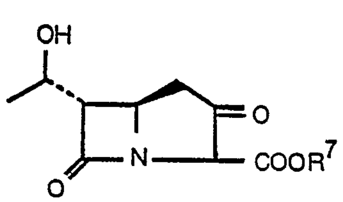

is prepared by cyclizing:

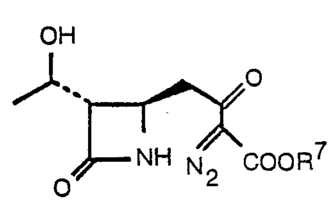

3. A process according to Claim 2, wherein the starting compound

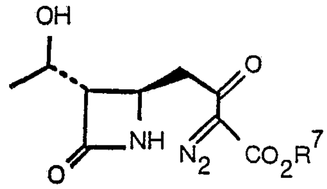

17

is prepared by
a) transforming

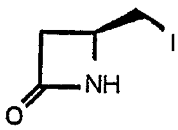

5

into protected

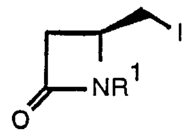

6

wherein $R^1$ is a protecting group, transforming 6 in a solvent with a carbanion generically represented by the following structure:

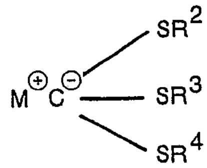

wherein M is a metal cation, and $R^2$, $R^3$ and $R^4$ are selected from alkyl, aryl or aralkyl, to

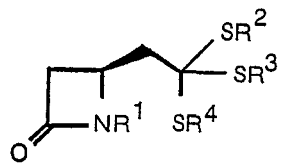

alkylation of 7 in a solvent at a temperature of from −100° to −20°C with a strong base followed by the addition of an equivalent to 10 fold excess of acetaldehyde to form

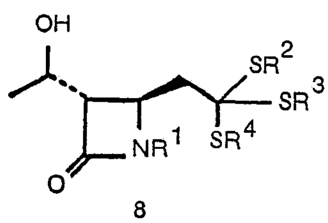

8

reacting 8 in an alcohol as solvent at a temperature of from 0 bis 80°C with a Lewis acid and inserting the hydroxyl protecting group $R^2$ to form

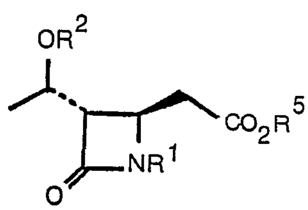

10

the value of $R^5$ being determined by the identity of the alcohol taken in reaction,
reducing 10 to form

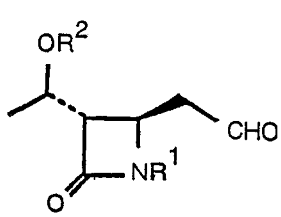

11

treating 11 in a solvent at a temperature of from −100 to 0°C for from 15 minutes to 2 hours in the presence of $LiCH_2CO_2R^6$; wherein $R^6$ is benzyl, p-methoxybenzyl or 2,4-dimethoxybenzyl, to form

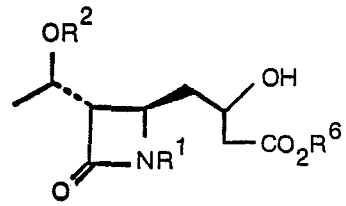

12

if desired, replacing $R^6$ by a more readily removable carboxyl protecting group, $R^7$, by selectively deblocking 12 by hydrogenation or hydrolysis, to form

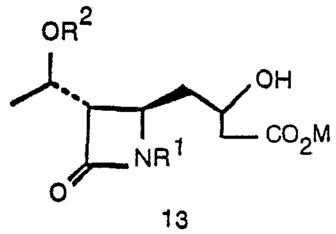

13

wherein M may be H, Na, K or ammonium and

treating the hydrogenation mixture with the chosen reagent calculated to establish the protecting group $R^7$ at a temperature of from 0° to 50°C for from 0.5 to 18 hours to form

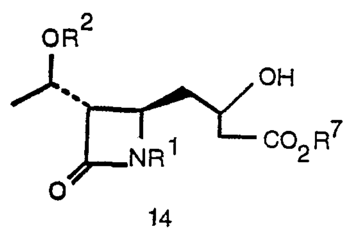

14

treating 14 in a solvent with an oxidizing system at a temperature of from −78°C to 25°C for from 5 min. to 8 hrs, to form

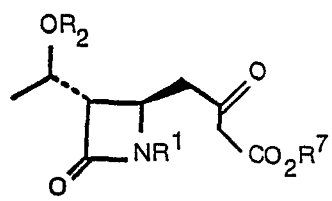

15

removing protecting groups $R^1$ and $R^2$ by acidic aqueous hydrolysis in a solvent in the presence of an acid at a temperature of from 0 to 100°C for from 2 to 18 hours, to form

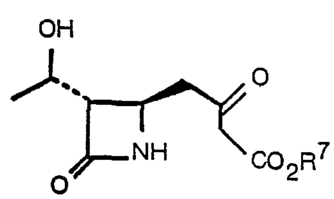

16

treating 16 in a solvent with an azide in the presence of a base for from 1 to 50 hours at 0—25°C, to form 17.

4. The compound:

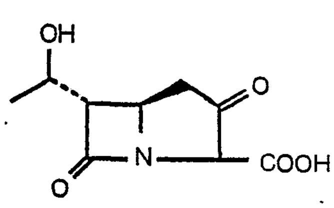

and its salt and protecting ester derivatives.

5. The compound:

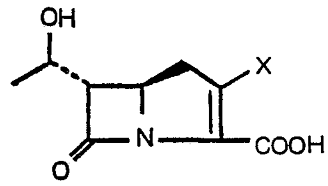

and its salt and protecting ester derivatives; wherein X is a organosulfonyloxy leaving group.

**Claims for the Contracting State: AT**

1. A process for preparing:

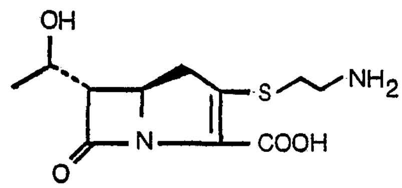

I

and its pharmaceutically acceptable salt and ester derivatives; comprising reacting:

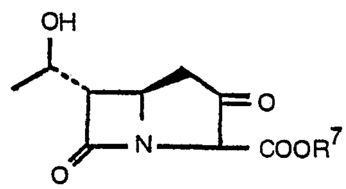

wherein $R^7$ is hydrogen or a protecting group with an acylating agent or a halogenating agent as an activating agent; reacting the obtained compound:

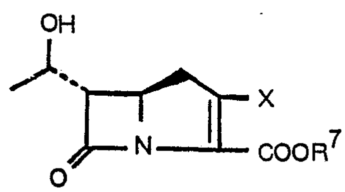

wherein X is a leaving group with $HSCH_2CH_2NHR^8$; wherein $R^8$ is hydrogen or a protecting group; and removal of the protecting groups $R^7$ and $R^8$.

2. A process according to Claim 1, wherein the starting compound

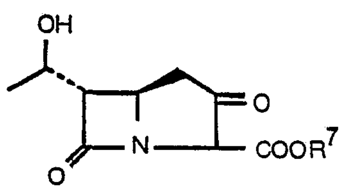 is prepared by cyclizing: 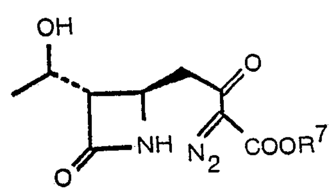

3. A process according to Claim 2, wherein the starting compound

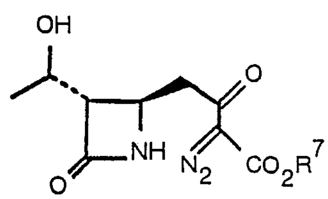

17

is prepared by

a) transforming

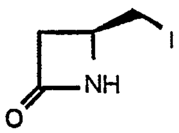

5

into protected

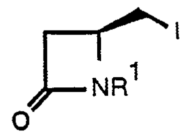

6

wherein $R^1$ is a protecting group, transforming 6 in a solvent with a carbanion generically represented by the following structure:

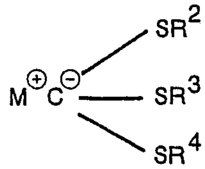

wherein M is a metal cation, and $R^2$, $R^3$ and $R^4$ are selected from alkyl, aryl or aralkyl, to

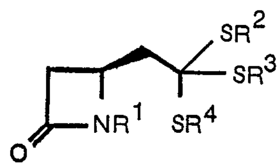

alkylation of 7 in a solvent at a temperature of from −100° to −20°C with a strong base followed by the addition of an equivalent to 10 fold excess of acetaldehyde to form

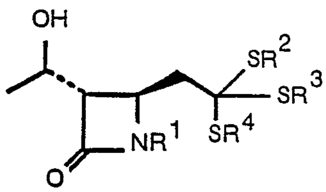

8

reacting 8 in an alcohol as solvent at a temperature of from 0 bis 80°C with a Lewis acid and inserting the hydroxyl protecting group $R^2$ to form

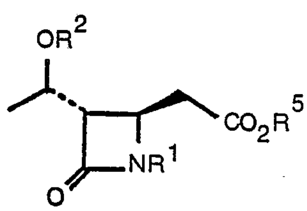

10

the value of $R^5$ being determined by the identity of the alcohol taken in reaction, reducing 10 to form

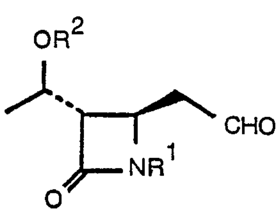

11

treating 11 in a solvent at a temperature of from −100°C to 0°C for from 15 minutes to 2 hours in the

presence of $LiCH_2CO_2R^6$; wherein $R^6$ is benzyl, p-methoxybenzyl or 2,4-dimethoxybenzyl, to form

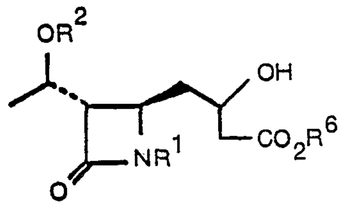

12

if desired, replacing $R^6$ by a more readily removable carboxyl protecting group, $R^7$, by selectively deblocking 12 by hydrogenation or hydrolysis, to form

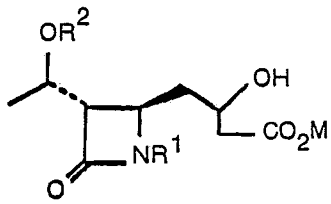

13

wherein M may be H, Na, K or ammonium and
treating the hydrogenation mixture with the chosen reagent calculated to establish the protecting group $R^7$ at a temperature of from 0° to 50°C for from 0.5 to 18 hours to form

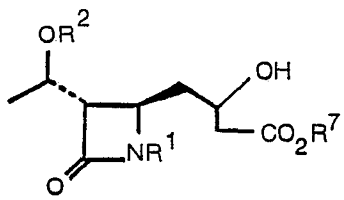

14

treating *14* in a solvent with an oxidizing system at a temperature of from −78°C to 25°C for from 5 min. to 8 hrs, to form

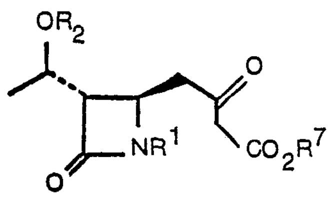

15

removing protecting groups $R^1$ and $R^2$ by acidic aqueous hydrolysis in a solvent in the presence of an acid at a temperature of from 0 to 100°C for from 2 to 18 hours, to form

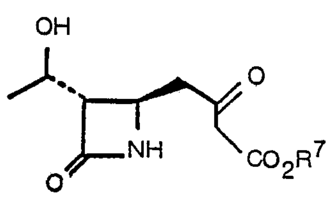

16

treating 16 in a solvent with an azide in the presence of a base for from 1 to 50 hours at 0—25°C, to form 17.

**Patentansprüche für die Vertragsstaaten: BE CH DE FR GB IT LU NL SE**

1. Ein Verfahren zur Herstellung von:

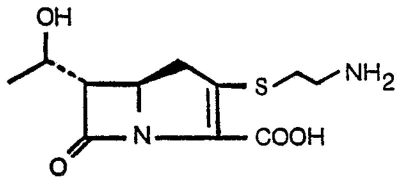

I

und von seinen pharmazeutisch annehmbaren Salz- und Esterderivaten; umfassend die Umsetzung von

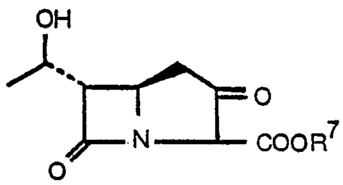

worin $R^7$ Wasserstoff oder eine Schutzgruppe ist, mit einem Acylierungsmittel oder einem Halogenierungsmittel als Aktivierungsmittel; die Umsetzung der erhaltenen Verbindung:

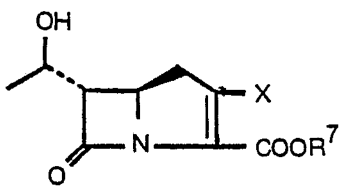

worin X eine austretende Gruppe ist, mit $HSCH_2CH_2NHR^8$, worin $R^8$ Wasserstoff oder eine Schutzgruppe ist; und das Entfernen der Schutzgruppen $R^7$ und $R^8$.

2. Ein Verfahren nach Anspruch 1, bei welchem die Ausgangsverbindung

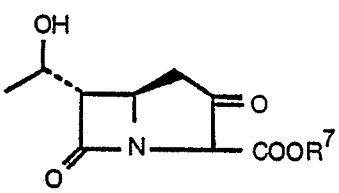 durch Cyclisieren von 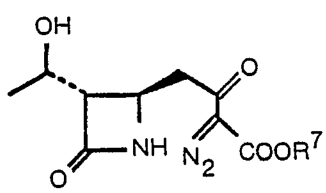

hergestellt wird.

3. Ein Verfahren nach Anspruch 2, bei welchem die Ausgangsverbindung

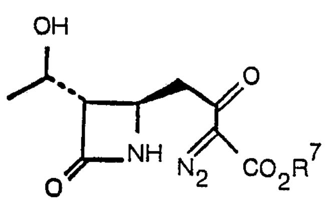

17

hergestellt wird durch Umwandlung von

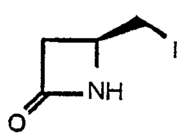 im geschütztes 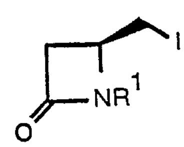

5 6

worin $R^1$ eine Schutzgruppe ist, Umwandlung von 6 in einem Lösungsmittel mit einem generisch durch die folgende Struktur repräsentierten Carbanion:

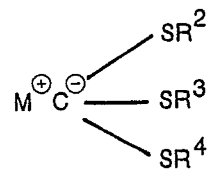

,

worin M ein Metallkation ist und $R^2$, $R^3$ und $R^4$ ausgewählt sind aus Alkyl, Aryl und Aralkyl, in die Verbindung

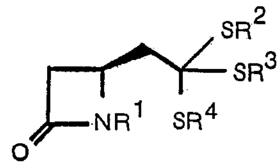

Alkylierung von 7 in einem Lösungsmittel bei einer Temperatur von —100° bis —20°C mit einer starken Base und nachfolgende Zugabe von einem Äquivalent bis zum 10-fachen Überschuß von Acetaldehyd, unter Bildung von

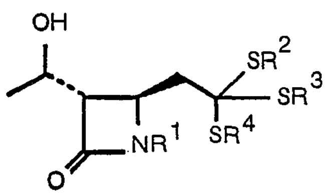

8

Umsetzung von 8 in einem Alkohol als Lösungsmittel bei einer Temperatur von 0 bis 80°C mit einer Lewis-Säure und Einführen der Schutzgruppe $R^2$ für die Hydroxylgruppe, unter Bildung von

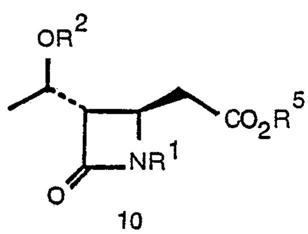

10

wobei die Natur von $R^5$ bestimmt wird von der Art des in die Reaktion eingegangenen Alkohols, Reduktion von 10 unter Bildung von

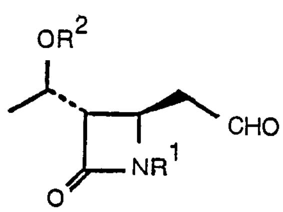

11

Behandlung von 11 in einem Lösungsmittel bei einer Temperatur von —100° bis 0°C während 15 Minuten bis 2 Stunden in Gegenwart von $LiCH_2CO_2R^6$, worin $R^6$ Benzyl, p-Methoxybenzyl oder 2,4-Dimethoxybenzyl ist, unter Bildung von

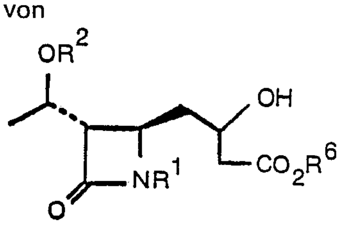

12

gewünschtenfalls Ersetzen von $R^6$ durch eine leichter entfernbare Schutzgruppe $R^7$ für die Carboxylgruppe durch selektives Entblockieren von 12 durch Hydrierung oder Hydrolyse unter Bildung von

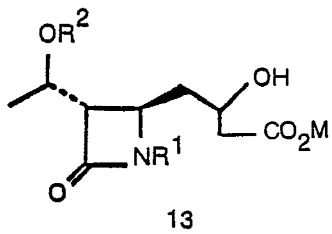

13

worin M H, Na, K oder Ammonium sein kann, und Behandlung des Hydrierungsgemisches mit dem gewählten Reagens, das die Schutzgruppe $R^7$ ausbilden soll, bei einer Temperatur von 0° bis 50°C während einer Dauer von 0,5 bis 18 Stunden, unter Bildung von

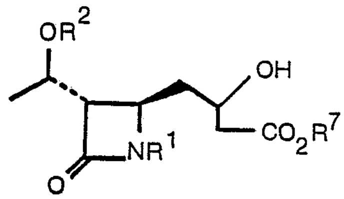

14

Behandlung von 14 in einem Lösungsmittel mit einem oxydierenden System bei einer Temperatur von —78° bis 25°C während 5 Minuten bis 8 Stunden unter Bildung von

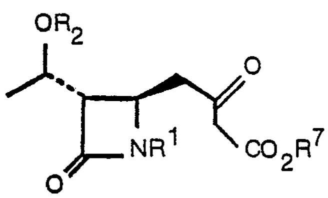

15

Entfernen der Schutzgruppen $R^1$ und $R^2$ durch saure wässerige Hydrolyse in einem Lösungsmittel in Gegenwart von einer Säure bei einer Temperatur von 0 bis 100°C während 2 bis 8 Stunden unter Bildung von

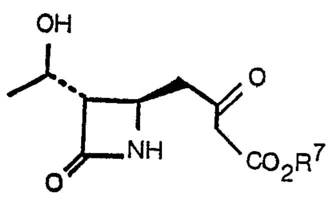

16

Behandlung von 16 in einem Lösungsmittel mit einem Azid in Gegenwart einer Base während 1 bis 50 Stunden bei 0 bis 25°C unter Bildung von 17.

4. Die Verbindung:

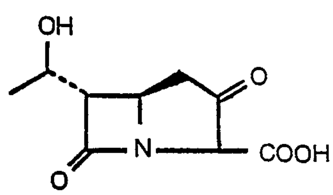

und deren Salz- und Schutzesterderivate.

5. Die Verbindung:

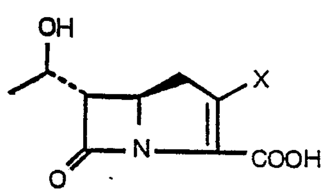

und deren Salz- und Schutzesterderivate, worin X eine austretende Organosulfonyloxygruppe ist.

**Patentansprüche für den Vertragsstaat: AT**

1. Ein Verfahren zur Herstellung von:

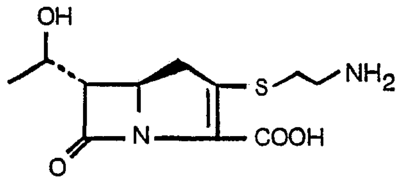

I

und von seinen pharmazeutisch annehmbaren Salz- und Esterderivaten; umfassend die Umsetzung von

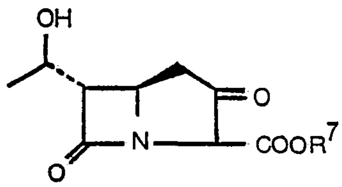

worin $R^7$ Wasserstoff oder eine Schutzgruppe ist, mit einem Acylierungsmittel oder einem Halogenierungsmittel als Aktivierungsmittel; die Umsetzung der erhaltenen Verbindung:

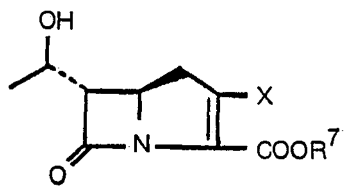

worin X eine austretende Gruppe ist, mit $HSCH_2CH_2NHR^8$, worin $R^8$ Wasserstoff oder eine Schutzgruppe ist; und das Entfernen der Schutzgruppen $R^7$ und $R^8$.

2. Ein Verfahren nach Anspruch 1, bei welchem die Ausgangsverbindung

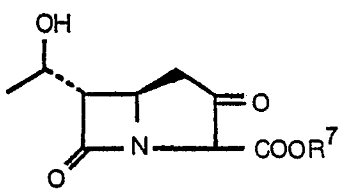

durch Cyclisieren von

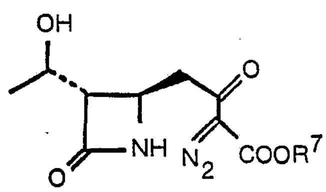

hergestellt wird.

3. Ein Verfahren nach Anspruch 2, bei welchem die Ausgangsverbindung

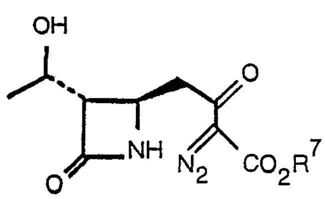

17

hergestellt wird
durch Umwandlung von

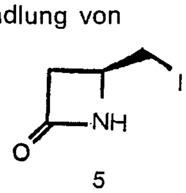

5

in geschütztes

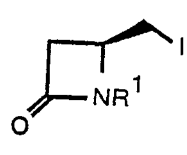

6

worin $R^1$ eine Schutzgruppe ist, Umwandlung von 6 in einem Lösungsmittel mit einem generisch durch die folgende Struktur repräsentierten Carbanion:

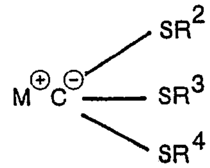

,

worin M ein Metallkation ist und $R^2$, $R^3$ und $R^4$ ausgewählt sind aus Alkyl, Aryl und Aralkyl, in die Verbindung

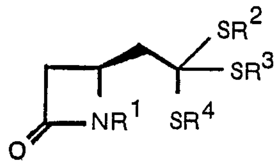

Alkylierung von 7 in einem Lösungsmittel bei einer Temperatur von $-100°$ bis $-20°C$ mit einer starken Base und nachfolgende Zugabe von einem Äquivalent bis zum 10-fachen Überschuß von Acetaldehyd, unter Bildung von

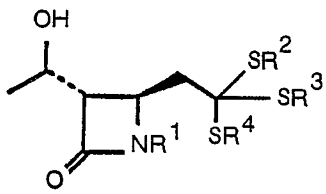

8

Umsetzung von 8 in einem Alkohol als Lösungsmittel bei einer Temperatur von 0 bis 80°C mit einer Lewis-Säure und Einführen der Schutzgruppe $R^2$ für die Hydroxylgruppe, unter Bildung von

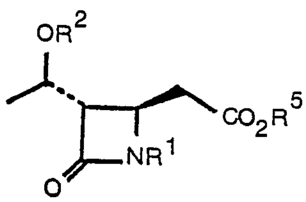

10

wobei die Natur von $R^5$ bestimmt wird von der Art des in die Reaktion eingegangenen Alkohols, Reduktion von 10 unter Bildung von

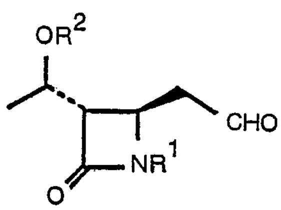

11

Behandlung von 11 in einem Lösungsmittel bei einer Temperatur $-100°$ bis 0°C während 15 Minuten bis 2 Stunden in Gegenwart von $LiCH_2CO_2R^6$, worin $R^6$ Benzyl, p-Methoxybenzyl oder 2,4-Dimethoxybenzyl ist, unter Bildung von

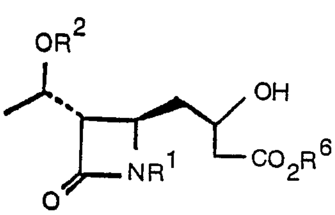

12

gewünschtenfalls Ersetzen von $R^6$ durch eine leichter entfernbare Schutzgruppe $R^7$ für die Carboxylgruppe durch selektives Entblockieren von 12 durch Hydrierung oder Hydrolyse unter Bildung von

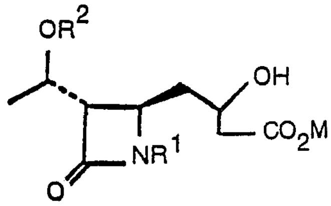

13

worin M H, Na, K oder Ammonium sein kann, und Behandlung des Hydrierungsgemisches mit dem gewählten Reagens, das die Schutzgruppe $R^7$ ausbilden soll, bei einer Temperatur von 0° bis 50°C während einer Dauer von 0,5 bis 18 Stunden, unter Bildung von

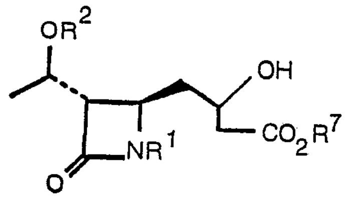

14

Behandlung von 14 in einem Lösungsmittel mit einem oxydierenden System bei einer Temperatur von −78°C bis 25°C während 5 Minuten bis 8 Stunden unter Bildung von

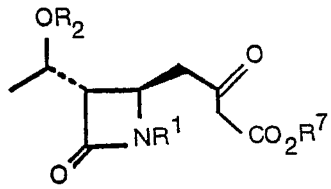

15

Entfernen der Schutzgruppen $R^1$ und $R^2$ durch saure wässerige Hydrolyse in einem Lösungsmittel in Gegenwart von einer Säure bei einer Temperatur von 0 bis 100°C während 2 bis 18 Stunden unter Bildung von

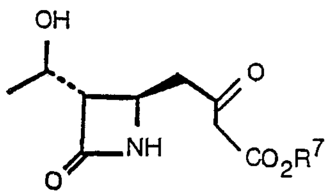

16

Behandlung von 16 in einem Lösungsmittel mit einem Azid in Gegenwart einer Base während 1 bis 50 Stunden bei 0 bis 25°C unter Bildung von 17.

**Revendications pour les Etats contractants**

1. Un procédé de préparation de:

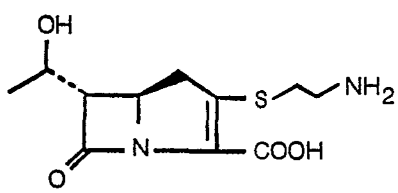

I

et ses sel et dérivés ester pharmaceutiquement acceptables;

comprenant la réaction de:

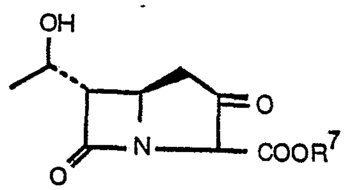

où $R^7$ est un hydrogène ou un groupe protecteur avec un agent acylant ou un agent halogénant comme agent d'activation, la réaction du composé obtenu:

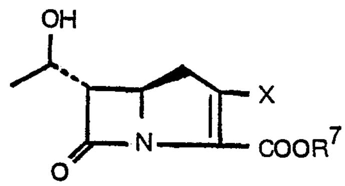

où X est un groupe partant avec $HSCH_2CH_2NR^8$ où $R^8$ est un hydrogène ou un groupe protecteur; et l'enlèvement des groupes protecteurs $R^7$ et $R^8$.

2. Un procédé selon la revendication 1, où le composé de départ

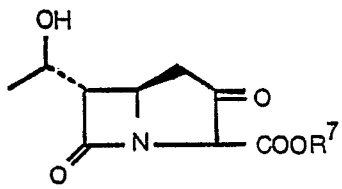

est préparé par cyclisation de:

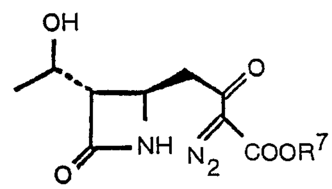

3. Un procédé selon la revendication 2, où le composé de départ

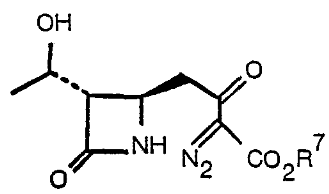

17

est préparé en

a) transformant

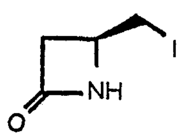

5

en un composé protégé

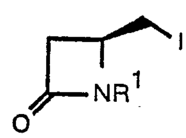

6

où $R^1$ est un groupe protecteur, en transformant 6 dans un solvant avec un carbanion représenté génériquement par la structure suivante:

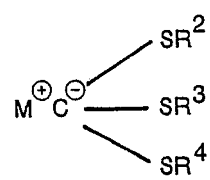

où M est un cation de métal, et $R^2$, $R^3$ et $R^4$ sont choisis parmi un alkyle, un aryle ou un aralkyle, en

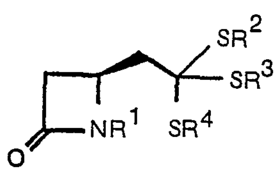

en alkylant 7 dans un solvant à une température comprise entre —100° et —20°C avec une base forte suivi par l'addition d'acétaldéhyde, entre un équivalent et un excès 10 fois pour former

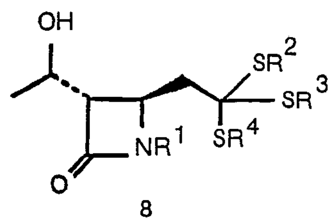

8

en faisant réagir 8 dans un alcool comme solvant à une température comprise entre 0 et 80°C avec un acide de Lewis et en introduisant le groupe protecteur hydroxyle $R^2$ pour former

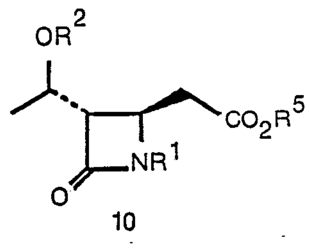

10

la valeur de $R^5$ étant déterminée par l'identité de l'alcool choisi pour la réaction, en réduisant 10 pour former

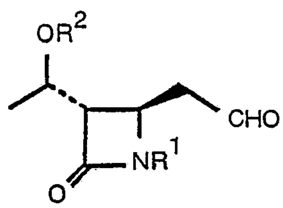

11

en traitant 11 dans un solvant à une température comprise entre —100° et 0°C pendant de 15 minutes à 2 heures en présence de $LiCH_2CO_2R^6$; où $R^6$ est un benzyle, un p-méthoxybenzyle ou un 2,4-diméthoxybenzyle, pour former

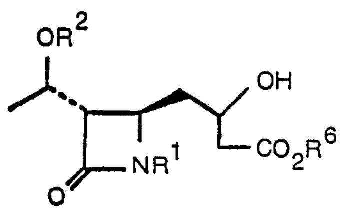

12

si on le désire, en remplaçant $R^6$ par un groupe protecteur carboxyle plus facilement enlevable, $R^7$, en débloquant sélectivement 12 par hydrogénation ou hydrolyse, pour former

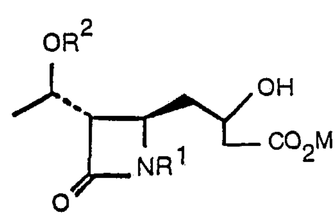

13

où M peut être H, Na, K ou un ammonium et en traitant le mélange d'hydrogénation avec le réactif choisi calculé pour mettre en place le groupe

protecteur $R^7$ à une température comprise entre 0° et 50°C pendant de 0,5 à 18 heures pour former

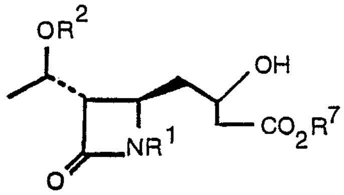

14

en traitant 14 dans un solvant avec un système oxydant à une température comprise entre −78°C et 25°C pendant de 5 min. à 8 hrs, pour former

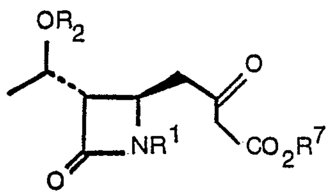

15

en enlevant les groupes protecteurs $R^1$ et $R^2$ par une hydrolyse aqueuse acide dans un solvant en présence d'un acide à une température comprise entre 0 et 100°C pendant de 2 à 18 heures, pour former

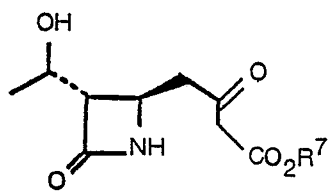

16

en traitant 16 dans un solvant avec un azide en présence d'une base pendant de 1 à 50 heures à 0—25°C, pour former 17.

4. Le composé:

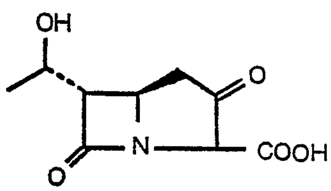

et ses sel et dérivés ester protecteurs.

5. Le composé:

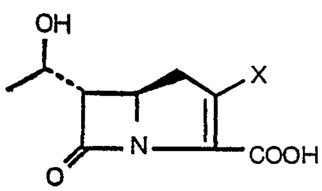

et ses sel et dérivés ester protecteurs; où X est un groupe partant organosulfonyloxy.

**Revendications pour L'Etat contractant: AT**

1. Un procédé de préparation de:

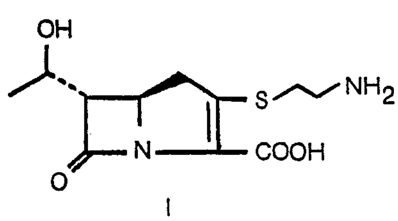

I

et ses sel et dérivés ester pharmaceutiquement acceptables; comprenant la réaction de:

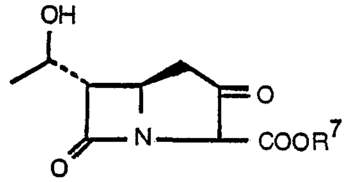

où $R^7$ est un hydrogène ou un groupe protecteur avec un agent acylant ou un agent halogénant comme agent d'activation, la réaction du composé obtenu:

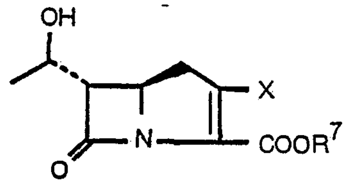

où X est un groupe partant avec $HSCH_2CH_2NHR^8$ où $R^8$ est un hydrogène ou un groupe protecteur; et l'enlèvement des groupes protecteurs $R^7$ et $R^8$.

2. Un procédé selon la revendication 1, où le composé de départ

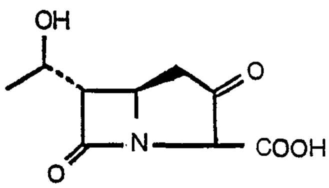

est préparé par cyclisation de:

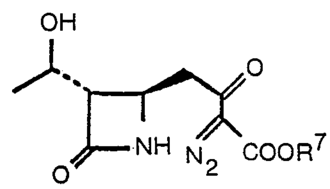

3. Un procédé selon la revendication 2, où le composé de départ

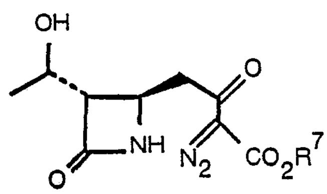

17

est préparé en

a) transformant

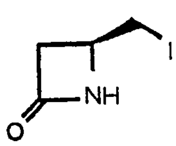

5

en un composé protégé

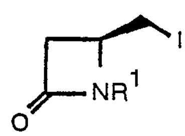

6

où $R^1$ est un groupe protecteur, en transformant 6 dans un solvant avec un carbanion représenté génériquement par la structure suivante:

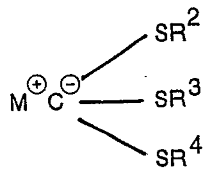

où M est un cation de métal, et $R^2$, $R^3$, et $R^4$ sont choisis parmi un alkyle, un aryle ou un aralkyle, en

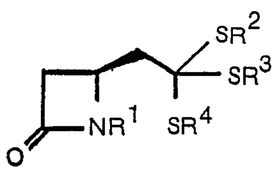

en alkylant 7 dans un solvant à une température comprise entre —100° et —20°C avec un base forte suivi par l'addition d'acétaldéhyde, entre un équivalent et un excès 10 fois pour former

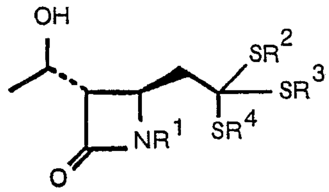

8

en faisant réagir 8 dans un alcool comme solvant à une température comprise entre 0 et 80°C avec un acide Lewis et en introduisant $R^2$ le groupe protecteur hydroxyle pour former

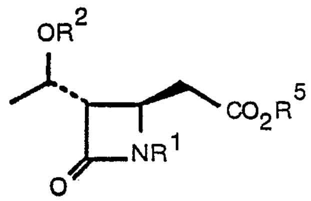

10

la valeur de $R^5$ étant déterminée par l'identité de l'alcool choisi pour la réaction, en réduisant 10 pour former

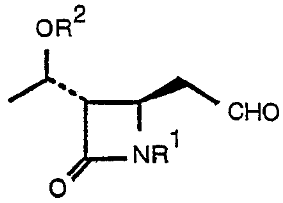

11

en traitant 11 dans un solvant à une température comprise entre —100° et 0°C pendant de 15 minutes à 2 heures en présence de $LiCH_2CO_2R^6$; où $R^6$ est un benzyle, un p-méthoxybenzyle ou un 2,4-diméthoxybenzyle, pour former

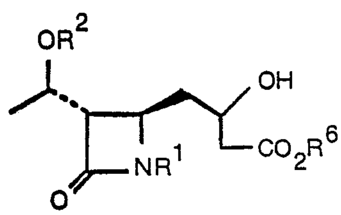

12

si on le désire, en remplaçant $R^6$ par un groupe protecteur carboxyle plus facilement enlevable, $R^7$, en débloquant sélectivement 12 par hydrogénation ou hydrolyse, pour former

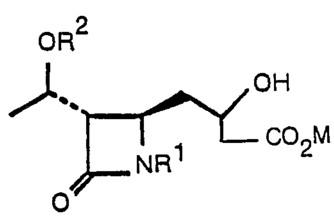

13

où M peut être H, Na, K ou un ammonium et

en traitant le mélange d'hydrogénation avec le réactif choisi calculé pour mettre en place le groupe

protecteur $R^7$ à une température comprise entre 0° et 50°C pendant de 0,5 à 18 heures pour former

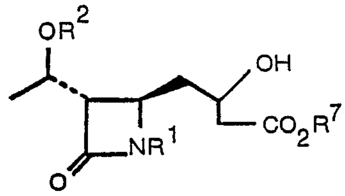

14

en traitant 14 dans un solvant avec un système oxydant à une température comprise entre —78°C et 25°C pendant de 5 min. à 8 hrs, pour former

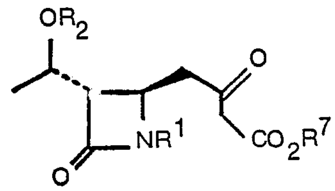

15

en enlevant les groupes protecteurs $R^1$ et $R^2$ par une hydrolyse aqueuse acide dans un solvant en présence d'un acide à une température comprise entre 0 et 100°C pendant de 2 à 18 heures, pour former

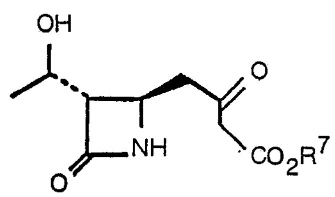

16

en traitant 16 dans un solvant avec un azide en présence d'une base pendant de 1 à 50 heures à 0—25°C, pour former 17.